(19) Europäisches Patentamt
European Patent Office
Office européen des brevets

(11) EP 3 669 856 A1

(12) EUROPÄISCHE PATENTANMELDUNG

(43) Veröffentlichungstag:
**24.06.2020 Patentblatt 2020/26**

(51) Int Cl.:
***A61K 6/09** (2006.01)* ***A61K 6/083** (2006.01)*

(21) Anmeldenummer: **18215766.9**

(22) Anmeldetag: **21.12.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Ivoclar Vivadent AG
9494 Schaan (LI)**

(72) Erfinder: **RIST, Kai
6800 Feldkirch (AT)**

(74) Vertreter: **Uexküll & Stolberg
Partnerschaft von
Patent- und Rechtsanwälten mbB
Beselerstraße 4
22607 Hamburg (DE)**

# (54) ZUSAMMENSETZUNGEN FÜR DIE HERSTELLUNG VON TRANSPARENTEN DENTALWERKSTÜCKEN MITTELS STEREOLITHOGRAPHIE

(57) Die vorliegende Erfindung betrifft eine polymerisierbare Zusammensetzung, die (a) mindestens eine radikalisch polymerisierbare Verbindung und (b) mindestens einen Initiator für die radikalische Polymerisation enthält, wobei die Zusammensetzung ferner (c) mindestens einen UV-Absorber und (d) mindestens einen optischen Aufheller enthält und als mindestens eine radikalisch polymerisierbare Verbindung (a) mindestens ein radikalisch polymerisierbares Oligomer und mindestens ein radikalisch polymerisierbares, polyfunktionelles Monomer enthält.

EP 3 669 856 A1

**Beschreibung**

Die vorliegende Erfindung betrifft Zusammensetzungen, die sich als Dentalwerkstoff besonders zur Herstellung von dentalen Werkstücken, insbesondere von orthodontischen Vorrichtungen, mit Hilfe von generativen Herstellungsverfahren, wie z.B. der Stereolithographie, eignen.

Fehlstellungen von Zähnen können nicht nur mit klassischen kieferorthopädischen Verfahren, z.B. mittels Keramikbrackets, sondern auch mit Hilfe von orthodontischen Apparaturen korrigiert werden. Beispielsweise ist die Korrektur von Zahnfehlstellung mit transparenten Kunststoffschienen, sog. Alignern, bereits seit den 1940ern bekannt. Die durchsichtigen Aligner bedecken komplett die Zähne und sollen 22 Stunden am Tag getragen und nur für das Zähneputzen oder Essen und Trinken herausgenommen werden. Bestandteil der Therapie sind häufig auch Verankerungselemente auf den Zähnen, sogenannte Attachments, die den Alignern Kraftansatz auf den Zähnen ermöglichen. Durch die Attachments können kontrollierte dreidimensionale Zahnbewegung durchgeführt werden, wie sie bspw. zur In- und Extrusion oder zur Derotation runder Zähne wie Eckzähnen oder Prämolaren benötigt werden. Auch für die optimale Feineinstellung der Zähne in der letzten Phase der Behandlung mit einer festen Spange wird häufig eine Schiene, ein sog. Positioner, empfohlen. Für die Herstellung von Positionern und Alignern werden herkömmlich von beiden Kiefern Gipsmodelle hergestellt und jeder einzelne Zahn aus dem Modell herausgetrennt. Danach werden die Zähne in Wachs in die optimale Stellung wieder aufgestellt, mit Silikon überzogen und in einem Spezialofen gehärtet.

Wie bei anderen dentaltechnischen Materialien sind auch bei orthodontischen Apparaturen gewisse Anforderungen an deren mechanischen Eigenschaften zu stellen. Beispielsweise ist bei orthodontischen Schienen, Alignern, Aligner-Attachments, Aligner-Attachments tragenden Positionern, Aufbissschienen (Splints), Transferschienen, Bohrschablonen, Prothesenwerkstoffen, Prothesenzähnen, Einprobekörpern, d.h. eine in einem Stück und einer Farbe ausgedruckte Prothese mit Zähnen zum Einprobieren und Kontrolle der Passung, und Zahnersatz eine hohe Bruchzähigkeit von Vorteil. Im Anwendungsfall müssen solche Werkstücke Verformungen ohne Bruch standhalten. Im Allgemeinen gilt für alle genannten Materialien, dass gleichzeitig eine hohe Festigkeit von Vorteil ist. Aligner-Attachments tragende Positioner werden z.B. über die Zähne geklipst, wofür sich die Positioner ohne Bruch verformen müssen. Da prozessbedingt Positioner und Attachments aus demselben Material gefertigt sein müssen, muss dieses Material gleichzeitig auch die Anforderungen erfüllen, die an das Attachment gestellt werden, nämlich ein ausreichend hohes Biegemodul und eine ausreichend hohe Biegefestigkeit aufweisen.

Zur Nutzung von computergestützten Fertigungsverfahren wäre es weiterhin wünschenswert, wenn sich solche Materialien mit Hilfe von generativen Verfahren verarbeiten ließen. Solche generativen Verfahren, die auch häufig unter dem Begriff "Rapid Prototyping" (RP) zusammengefasst werden, werden in jüngerer Zeit vermehrt zur Herstellung von dentalen Werkstücken eingesetzt. Hierunter werden Fertigungsverfahren verstanden, bei denen aus computergestützten Konstruktionsdaten (CAD-Daten) dreidimensionale Modelle oder Bauteile schichtweise oder kontinuierlich hergestellt werden (A. Gebhardt, Vision of Rapid Prototyping, Ber. DGK 83 (2006) 7-12). Dabei handelt es sich um Verfahren, wie z.B. die Stereolithographie (SL), selektives Lasersintern (SLS), 3-D-Printing, Fused Deposition Modelling (FDM), Ink-Jet-Printing (IJP), 3D-Plotting, Multi-Jet Modelling (MJM), Solid Freeform Fabrication (SFF), Laminated Object Manufacturing (LOM), Laser Powder Forming (LPF), mit denen sich Modelle, Bau- oder Formteile auch in Kleinserie kostengünstig herstellen lassen (A. Gebhardt, Generative Fertigungsverfahren, 3. Aufl., Carl Hanser Verlag, München 2007, 77ff.). Bei der Stereolithographie wird ein Formteil auf der Basis von CAD-Daten schichtweise aus einem flüssigen und härtbaren Monomerharz aufgebaut (A. Beil, Fertigung von Mikro-Bauteilen mittels Stereolithographie, Düsseldorf 2002, VDI-Verlag 3 ff.).

Damit müssen diese Materialien zwingend lichthärtend sein und zudem eine niedrige Viskosität aufweisen, da sie ansonsten in einfachen Geräten ohne Heizung und Rakel nicht ohne weiteres verarbeitet werden können.

Des Weiteren sind insbesondere hohe Anforderungen an die optischen Eigenschaften der Materialien zu stellen. Speziell für Schienen, Aligner, Positioner und Aligner-Attachments ist eine möglichst geringe Eigenfarbe bei gleichzeitig hoher Transparenz der gehärteten Zusammensetzung besonders wünschenswert, um eine möglichst unscheinbare und somit ästhetische orthodontische Apparatur bereitzustellen. Dies gestaltet sich in der Praxis jedoch häufig schwierig, da Additive, wie z.B. Füllstoffe, die der Zusammensetzung zur Verbesserung der mechanischen Eigenschaften zugesetzt werden, oder UV-Blocker, die der Zusammensetzung zur besseren Kontrolle der Eindringtiefe des zur Härtung eingesetzten Lichts und damit zur Verbesserung der Präzision der stereolithographischen Herstellung zugesetzt werden, nicht selten eine gewisse, wahrnehmbare Eigenfarbe aufweisen.

Der Erfindung liegt somit die Aufgabe zugrunde, Materialien zur Verfügung zu stellen, die die oben genannten Anforderungen erfüllen. Insbesondere sollen die Materialien lichthärtend sein und eine niedrige Viskosität aufweisen, so dass sie in einem generativen Fertigungsverfahren, insbesondere mit Hilfe eines stereolithographischen Druckers, zu verarbeiten sind. Zudem sollen sich die gehärteten Materialien insbesondere durch eine geringe Eigenfarbe und eine hohe Transparenz auszeichnen. Auch ausreichende mechanische Eigenschaften, wie eine hohe Bruchzähigkeit, insbesondere eine hohe Randfaserdehnung, bei gleichzeitig hoher Biegefestigkeit und hohem Biegemodul, sind erstrebenswert.

Erfindungsgemäß wird diese Aufgabe durch polymerisierbare Zusammensetzungen gelöst, die (a) mindestens eine radikalisch polymerisierbare Verbindung und (b) mindestens einen Initiator für die radikalische Polymerisation enthalten.

Die erfindungsgemäßen Zusammensetzungen sind dadurch gekennzeichnet, dass sie ferner (c) mindestens einen UV-Absorber und (d) mindestens einen optischen Aufheller enthalten.

Die mindestens eine radikalisch polymerisierbare Verbindung (a) umfasst ein radikalisch polymerisierbares Oligomer und ein radikalisch polymerisierbares Monomer, das polyfunktionell ist.

Vorzugsweise ist das radikalisch polymerisierbare Oligomer ausgewählt aus der Gruppe bestehend aus (i) aliphatischen Urethan(meth)acrylat-Oligomeren, (ii) Epoxy(meth)acrylat-Oligomeren und (iii) Polyether-Urethan(meth)acrylat-Oligomeren, wobei die aliphatischen Urethan(meth)acrylat-Oligomeren vorzugsweise Polyether-Urethan(meth)acrylat-Oligomere oder Polyester-Urethan(meth)acrylat-Oligomere sind. Besonders bevorzugt ist das radikalisch polymerisierbare Oligomer ausgewählt aus der Gruppe bestehend aus di- oder tetrafunktionellen aliphatischen Urethan(meth)acrylat-Oligomeren und difunktionellen Epoxy(meth)acrylat-Oligomeren.

In einer ersten besonders bevorzugten Ausführungsform sind die erfindungsgemäßen Zusammensetzungen dadurch gekennzeichnet, dass das radikalisch polymerisierbare Oligomer ein Oligomer (a1) ist, das durch Polymerisation von 2-Hydroxyethyl(meth)acrylat, 1-Isocyanato-3-isocyanatomethyl-3,5,5-trimethylcyclohexan, ε-Caprolacton und 2-(2-Hydroxyethoxy)ethanol erhältlich ist.

Vorzugsweise weist das Oligomer (a1) eine lineare Struktur auf, wobei die beiden Kettenenden jeweils durch 2-Hydroxyethyl(meth)acrylateinheiten gebildet werden, so dass pro Oligomer 2 freie Acrylat- oder Methacrylatgruppen zur radikalischen Polymerisation mit dem polyfunktionellen Monomer (b) und ggf. weiteren Bestandteilen zur Verfügung stehen. Des Weiteren ist es bevorzugt, dass die Hydroxygruppen von den beiden endständigen 2-Hydroxyethyl(meth)acrylatresten zur Kettenmitte zeigen und jeweils mit einer der beiden Cyanogruppen von 1-Isocyanato-3-isocyanatomethyl-3,5,5-trimethylcyclohexan eine Urethanbindung ausbilden. Die jeweils andere Cyanogruppe des 1-Isocyanato-3-isocyanatomethyl-3,5,5-trimethylcyclohexans bildet mit Hydroxygruppen eines Kettenmittelsegments eine Urethanbindung aus, wobei das Kettenmittelsegment durch Polymerisation von ε-Caprolacton und 2-(2-Hydroxyethoxy)ethanol oder durch Polymerisation von 1-Isocyanato-3-isocyanatomethyl-3,5,5-trimethylcyclohexan, ε-Caprolacton und 2-(2-Hydroxyethoxy)ethanol erhältlich ist.

In einer bevorzugten Ausführungsform kann das Oligomer (a1) durch folgende Strukturformel (I)

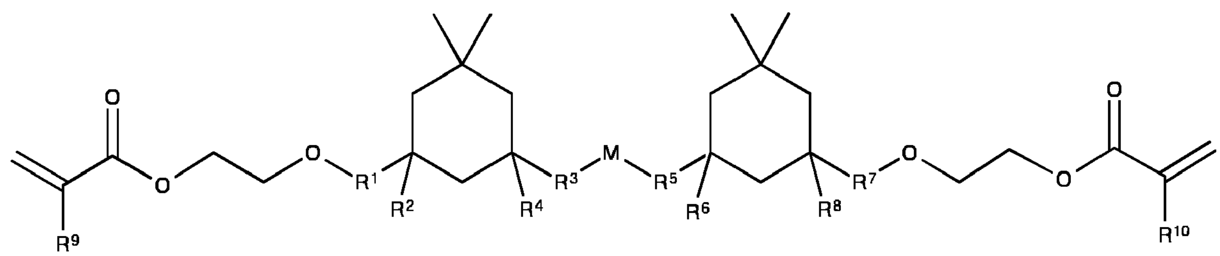

(I)

wiedergegeben werden, in der

$R^1$ und $R^5$ jeweils -C(=O)-NH- oder -C(=O)-NH-$CH_2$- sind,
$R^3$ und $R^7$ jeweils -NH-C(=O)- oder $CH_2$-NH-C(=O)- sind,
$R^2$, $R^4$, $R^6$ und $R^8$ jeweils H oder $CH_3$ sind,
$R^9$ und $R^{10}$ jeweils H oder $CH_3$ sind,
wobei,
wenn $R^1$ -C(=O)-NH- ist, dann ist $R^2$ H, $R^3$ -$CH_2$-NH-C(=O)- und $R^4$ $CH_3$, und
wenn $R^1$ -C(=O)-NH-$CH_2$- ist, dann ist $R^2$ $CH_3$, $R^3$ -NH-C(=O)- und $R^4$ H,
wobei $R^1$, $R^3$, $R^5$ und $R^7$ jeweils von links nach rechts in die Kette der Struktur (I) eingefügt sind, und
M ein Kettenmittelsegment ist, das durch Polymerisation von ε-Caprolacton und 2-(2-Hydroxyethoxy)ethanol oder durch Polymerisation von 1-Isocyanato-3-isocyanatomethyl-3,5,5-trimethylcyclohexan, ε-Caprolacton und 2-(2-Hydroxyethoxy)ethanol erhältlich ist.

Vorzugsweise kann das Oligomer (a1) durch folgende Strukturformel (II)

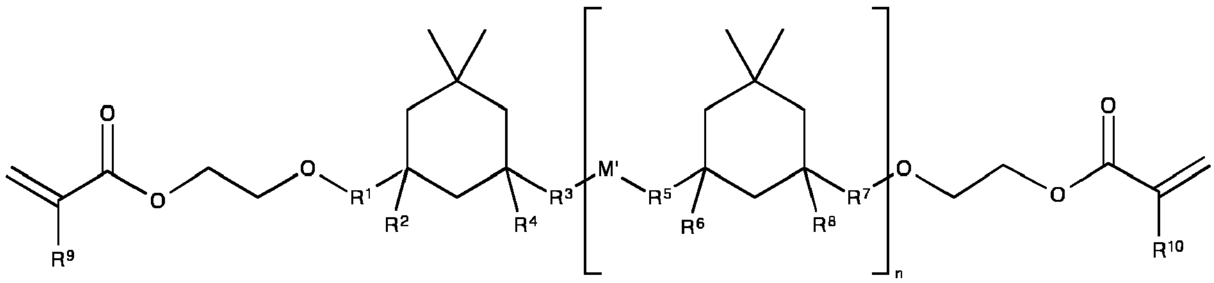

(II)

wiedergegeben werden, in der

$R^1$ bis $R^{10}$ die obigen Bedeutungen haben,

M' ein Kettenmittelsegment ist, das durch Polymerisation von ε-Caprolacton und 2-(2-Hydroxyethoxy)ethanol erhältlich ist, und

n eine ganze Zahl von 1 bis 10, vorzugsweise 1 bis 7, besonders bevorzugt 1 bis 5, wie etwa 1 bis 4 oder 2 bis 3, und insbesondere 1 oder 2 und ganz besonders bevorzugt 1 ist.

In einer Ausführungsform wird das Kettenmittelsegment M' in obiger Formel (II) durch Strukturformel (III) wiedergegeben

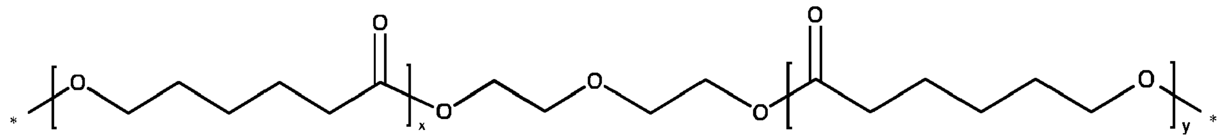

(III),

wobei x eine Zahl von 1 bis 20, vorzugsweise 1 bis 15, besonders bevorzugt 1 bis 5, wie etwa 2 bis 3, ist und

y eine Zahl von 1 bis 20, vorzugsweise 1 bis 15, besonders bevorzugt 1 bis 5, wie etwa 2 bis 3, ist.

Dabei ist es besonders bevorzugt, dass $R^9$ und $R^{10}$ jeweils die gleiche Bedeutung haben. Insbesondere sind $R^9$ und $R^{10}$ beide H, so dass Oligomer (a1) ausgewählt ist aus der Gruppe bestehend aus Oligomeren, die durch Polymerisation von 2-Hydroxyethylacrylat, 1-Isocyanato-3-isocyanatomethyl-3,5,5-trimethylcyclohexan, ε-Caprolacton und 2-(2-Hydroxyethoxy)ethanol erhältlich sind.

In einer zweiten besonders bevorzugten Ausführungsform ist das Oligomer ein Oligomer (a2), das durch Polymerisation von 2-Hydroxyethyl(meth)acrylat, 1-Isocyanato-3-isocyanatomethyl-3,5,5-trimethylcyclohexan, Adipinsäure und Ethylenglykol erhältlich ist.

In einer bevorzugten Ausführungsform kann das Oligomer (a2) durch obige Strukturformel (I) wiedergegeben werden, in der $R^1$ bis $R^{10}$ die obige Bedeutung aufweisen und M ein Kettenmittelsegment ist, das durch Polymerisation von Adipinsäure und Ethylenglykol oder durch Polymerisation von 1-Isocyanato-3-isocyanatomethyl-3,5,5-trimethylcyclohexan, Adipinsäure und Ethylenglykol erhältlich ist.

Vorzugsweise kann das Oligomer (a2) durch obige Strukturformel (II) wiedergegeben werden, in der $R^1$ bis $R^{10}$ die obigen Bedeutungen haben und M' ein Kettenmittelsegment ist, das durch Polymerisation von Adipinsäure und Ethylenglykol erhältlich ist, und n eine ganze Zahl von 1 bis 10, vorzugsweise 1 bis 7, besonders bevorzugt 1 bis 5, wie etwa 1 bis 4 oder 2 bis 3, und insbesondere 1 oder 2 und ganz besonders bevorzugt 1 ist.

In einer Ausführungsform von Oligomer (a2) wird das Kettenmittelsegment M' in obiger Formel (II) durch Strukturformel (IV) wiedergegeben

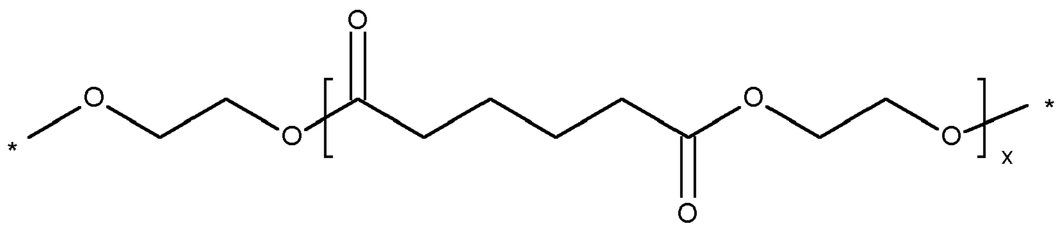

(IV),

wobei x eine Zahl von 1 bis 20, vorzugsweise 1 bis 15, besonders bevorzugt 1 bis 5, wie etwa 2 bis 4, ist.

Dabei ist es besonders bevorzugt, dass $R^9$ und $R^{10}$ jeweils die gleiche Bedeutung haben. Insbesondere sind $R^9$ und $R^{10}$ beide H, so dass Oligomer (a2) ausgewählt ist aus der Gruppe bestehend aus Oligomeren, die durch Polymerisation von 2-Hydroxyethylacrylat, 1-Isocyanato-3-isocyanatomethyl-3,5,5-trimethylcyclohexan, Adipinsäure und Ethylenglykol erhältlich sind.

In einer dritten besonders bevorzugten Ausführungsform ist das Oligomer ein Oligomer (a3), das durch Polymerisation von 2-Hydroxyethyl(meth)acrylat, 1-Isocyanato-3-isocyanatomethyl-3,5,5-trimethylcyclohexan und Tetrahydrofuran erhältlich ist.

In einer bevorzugten Ausführungsform kann das Oligomer (a3) durch obige Strukturformel (I) wiedergegeben werden, in der $R^1$ bis $R^{10}$ die obige Bedeutung aufweisen und M ein Kettenmittelsegment ist, das durch Polymerisation von Tetrahydrofuran oder durch Polymerisation von 1-Isocyanato-3-isocyanatomethyl-3,5,5-trimethylcyclohexan und Tetrahydrofuran erhältlich ist.

Vorzugsweise kann das Oligomer (a3) durch obige Strukturformel (II) wiedergegeben werden, in der $R^1$ bis $R^{10}$ die obigen Bedeutungen haben und M' ein Kettenmittelsegment ist, das durch Polymerisation von Tetrahydrofuran erhältlich ist, und n eine ganze Zahl von 1 bis 10, vorzugsweise 1 bis 7, besonders bevorzugt 1 bis 5, wie etwa 1 bis 4 oder 2 bis 3, und insbesondere 1 oder 2 und ganz besonders bevorzugt 1 ist.

In einer Ausführungsform von Oligomer (a3) wird das Kettenmittelsegment M' in obiger Formel (II) durch Strukturformel (V) wiedergegeben

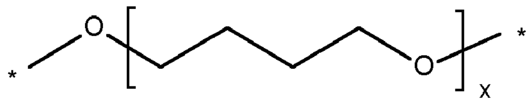

(V),

wobei x eine Zahl von 1 bis 20, vorzugsweise 1 bis 15, besonders bevorzugt 5 bis 15, wie etwa 8 bis 12, ist.

Dabei ist es besonders bevorzugt, dass $R^9$ und $R^{10}$ jeweils die gleiche Bedeutung haben. Insbesondere sind $R^9$ und $R^{10}$ beide H, so dass Oligomer (a3) ausgewählt ist aus der Gruppe bestehend aus Oligomeren, die durch Polymerisation von 2-Hydroxyethylacrylat, 1-Isocyanato-3-isocyanatomethyl-3,5,5-trimethylcyclohexan und Tetrahydrofuran erhältlich sind. Alternativ sind $R^9$ und $R^{10}$ beide $CH_3$, so dass Oligomer (a3) ausgewählt ist aus der Gruppe bestehend aus Oligomeren, die durch Polymerisation von 2-Hydroxyethylmethacrylat, 1-Isocyanato-3-isocyanatomethyl-3,5,5-trimethylcyclohexan und Tetrahydrofuran erhältlich sind.

In einer vierten besonders bevorzugten Ausführungsform ist das Oligomer ein Oligomer (a4), das durch Polymerisation von 2-Hydroxyethyl(meth)acrylat, 1-Isocyanato-3-isocyanatomethyl-3,5,5-trimethylcyclohexan und Ethylenglykol erhältlich ist.

In einer bevorzugten Ausführungsform kann das Oligomer (a4) durch obige Strukturformel (I) wiedergegeben werden, in der $R^1$ bis $R^{10}$ die obige Bedeutung aufweisen und M ein Kettenmittelsegment ist, das durch Polymerisation von Ethylenglykol oder durch Polymerisation von 1-Isocyanato-3-isocyanatomethyl-3,5,5-trimethylcyclohexan und Ethylenglykol erhältlich ist. Vorzugsweise kann das Oligomer (a4) durch obige Strukturformel (II) wiedergegeben werden, in der $R^1$ bis $R^{10}$ die obigen Bedeutungen haben und M' ein Kettenmittelsegment ist, das durch Polymerisation von Ethylenglykol erhältlich ist, und n eine ganze Zahl von 1 bis 10, vorzugsweise 1 bis 7, besonders bevorzugt 1 bis 5, wie etwa 1 bis 4 oder 2 bis 3, und insbesondere 1 oder 2 und ganz besonders bevorzugt 1 ist.

In einer Ausführungsform von Oligomer (a4) wird das Kettenmittelsegment M' in obiger Formel (II) durch Strukturformel (VI) wiedergegeben

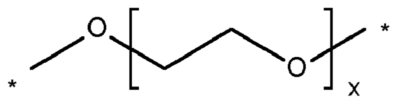

(VI),

wobei x eine Zahl von 1 bis 20, vorzugsweise 1 bis 15, besonders bevorzugt 5 bis 15, wie etwa 6 bis 11, wie z.B. 7 bis 10 ist.

Dabei ist es besonders bevorzugt, dass $R^9$ und $R^{10}$ jeweils die gleiche Bedeutung haben. Insbesondere sind $R^9$ und $R^{10}$ beide $-CH_3$, so dass Oligomer (a4) ausgewählt ist aus der Gruppe bestehend aus Oligomeren, die durch Polymerisation von 2-Hydroxyethylmethacrylat, 1-Isocyanato-3-isocyanatomethyl-3,5,5-trimethylcyclohexan und Ethylenglykol erhältlich sind.

In einer fünften besonders bevorzugten Ausführungsform ist das Oligomer ein Oligomer (a5), das durch Polymerisation von 2-Hydroxyethyl(meth)acrylat, 2,2,4-Trimethylhexandiol-diisocyanat und Ethylenglykol erhältlich ist.

In einer bevorzugten Ausführungsform kann das Oligomer (a5) durch die folgende Strukturformel (VII)

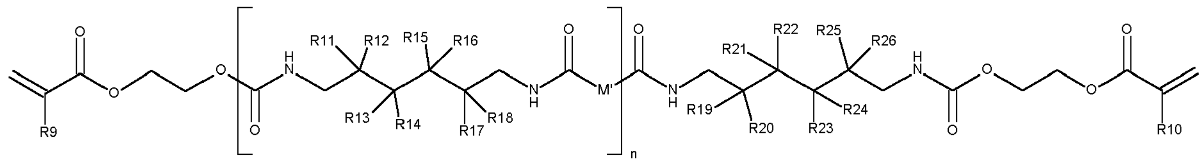

(VII)

wiedergegeben werden, in der

$R^9$ und $R^{10}$ die obige Bedeutung aufweisen,

$R^{11}$ bis $R^{26}$ H oder $CH_3$ sind,

wobei

entweder $R^{11}$, $R^{12}$ und $R^{15}$ $CH_3$ sind und $R^{13}$, $R^{14}$, $R^{16}$, $R^{17}$ und $R^{18}$ H sind,
oder $R^{13}$, $R^{17}$ und $R^{18}$ $CH_3$ sind und $R^{11}$, $R^{12}$, $R^{14}$, $R^{15}$ und $R^{16}$ H sind,

und

entweder $R^{19}$, $R^{20}$ und $R^{23}$ $CH_3$ sind und $R^{21}$, $R^{22}$, $R^{24}$, $R^{25}$ und $R^{26}$ H sind,
oder $R^{21}$, $R^{25}$ und $R^{26}$ $CH_3$ sind und $R^{19}$, $R^{20}$, $R^{22}$, $R^{23}$ und $R^{24}$ H sind,

M' ein Kettenmittelsegment ist, das durch Polymerisation von Ethylenglykol erhältlich ist, und

n eine ganze Zahl von 1 bis 10, vorzugsweise 1 bis 7, besonders bevorzugt 1 bis 5, wie etwa 1 bis 4 oder 2 bis 3, und insbesondere 1 oder 2 ist.

In einer Ausführungsform von Oligomer (a5) wird das Kettenmittelsegment M' in obiger Formel (VII) durch Strukturformel (VIII) wiedergegeben

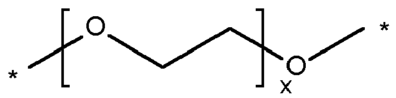

(VIII),

wobei x eine Zahl von 1 bis 20, vorzugsweise 1 bis 15, besonders bevorzugt 5 bis 15, wie etwa 6 bis 11, wie z.B. 6 bis 9 ist. Dabei ist es besonders bevorzugt, dass $R^9$ und $R^{10}$ jeweils die gleiche Bedeutung haben. Insbesondere sind $R^9$

und $R^{10}$ beide $-CH_3$, so dass Oligomer (a5) ausgewählt ist aus der Gruppe bestehend aus Oligomeren, die durch Polymerisation von 2-Hydroxyethylmethacrylat, 2,2,4-Trimethylhexandiol-diisocyanat und Ethylenglykol erhältlich sind.

In einer sechsten besonders bevorzugten Ausführungsform ist das Oligomer ein Oligomer (a6), das durch Polymerisation von 2-Hydroxyethyl(meth)acrylat, 2,2,4-Trimethylhexandiol-diisocyanat und Tetrahydrofuran erhältlich ist.

In einer bevorzugten Ausführungsform kann das Oligomer (a6) durch die obige Strukturformel (VII) wiedergegeben werden, in der $R^9$ bis $R^{26}$ die obige Bedeutung aufweisen, M' ein Kettenmittelsegment ist, das durch Polymerisation von Tetrahydrofuran erhältlich ist, und n eine ganze Zahl von 1 bis 10, vorzugsweise 1 bis 7, besonders bevorzugt 1 bis 5, wie etwa 1 bis 4 oder 2 bis 3, und insbesondere 1 oder 2 ist.

In einer Ausführungsform von Oligomer (a6) wird das Kettenmittelsegment M' in obiger Formel (VII) durch Strukturformel (IX) wiedergegeben

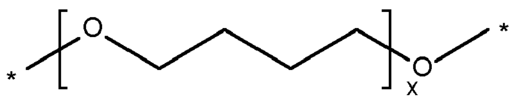

(IX),

wobei x eine Zahl von 1 bis 20, vorzugsweise 1 bis 15, besonders bevorzugt 5 bis 15, wie etwa 7 bis 12, wie z.B. 8 bis 11 ist. Dabei ist es besonders bevorzugt, dass $R^9$ und $R^{10}$ jeweils die gleiche Bedeutung haben. Insbesondere sind $R^9$ und $R^{10}$ beide $-CH_3$, so dass Oligomer (a6) ausgewählt ist aus der Gruppe bestehend aus Oligomeren, die durch Polymerisation von 2-Hydroxyethylmethacrylat, 2,2,4-Trimethylhexandiol-diisocyanat und Tetrahydrofuran erhältlich sind.

In einer siebten besonders bevorzugten Ausführungsform ist das Oligomer ein Oligomer (a7), das ein aliphatisches tetrafunktionelles Urethanmethacrylat-Oligomer mit einer Molmasse von etwa 7000 bis 9000 g/mol, insbesondere etwa 8000 g/mol, gemessen mittels GPC, ist und vorzugsweise in Form einer Mischung mit Triethylenglycoldimethacrylat vorliegt, wobei die Mischung vorzugsweise 80 bis 95 Gew.-%, insbesondere etwa 90 Gew.-%, von Oligomer (a7) umfasst. Beispielsweise ist Oligomer (a7) Miramer U3400 NT, erhältlich von Miwon Specialty Chemical Co., Ltd.

In einer achten besonders bevorzugten Ausführungsform ist das Oligomer (a) ein Oligomer (a8), das ein difunktionelles Epoxyacrylat-Oligomer mit einer Molmasse von etwa 6000 bis 7000 g/mol, wie etwa 6600 g/mol, wie z.B. Photocryl E207, erhältlich von Miwon Specialty Chemical Co., Ltd.

Ganz besonders bevorzugt enthält die erfindungsgemäße Zusammensetzung als Oligomer das oben beschriebene Oligomer (a1).

Das radikalisch polymerisierbare Oligomer weist vorzugsweise eine Molmasse (ausgedrückt als Zahlenmittel Mn, das insbesondere im Fall von Polyester-Urethan-Oligomeren über die Hydroxylzahl des bei der Synthese verwendeten Polyesterdiols sowie die Molmassen des für die Synthese verwendeten Isocyanats und (Meth)acrylats berechnet werden kann) von mehr als 500 g/mol, insbesondere 500 bis 10000 g/mol, wie z.B. 500 bis 8000 g/mol oder 1000 bis 7000 g/mol oder 1000 bis 5000 g/mol, besonders bevorzugt 500 bis 3000 g/mol auf, wie z.B. 1200 bis 2500 g/mol. Des Weiteren ist es bevorzugt, dass das radikalisch polymerisierbare Oligomer eine Viskosität von 5 bis 150, vorzugsweise 10 bis 50, insbesondere 20 bis 25 Pas, gemessen mittels Rotationsviskosimetrie bei 50°C, aufweist.

Das radikalisch polymerisierbare Oligomer liegt, bezogen auf das Gesamtgewicht der Zusammensetzung, vorzugsweise in einer Menge von 25 bis 60 Gew.-%, insbesondere 30 bis 55 Gew.-%, wie z.B. 32 bis 50 Gew.-%, vor. Für den Fall, dass die erfindungsgemäße Zusammensetzung einen Füllstoff (e) enthält, wie unten beschrieben ist, beträgt die Menge an radikalisch polymerisierbarem Oligomer vorzugsweise 38 bis 42 Gew.-%. Für den Fall, dass die erfindungsgemäße Zusammensetzung keinen Füllstoff enthält, beträgt die Menge an radikalisch polymerisierbarem Oligomer vorzugsweise 40 bis 55 Gew.-%, insbesondere 45 bis 50 Gew.-%.

Es wurde überraschend gefunden, dass die Verwendung eines oben beschriebenen Oligomers in Kombination mit einem polyfunktionellen Monomeren und einem Initiator (b) Zusammensetzungen ergibt, die lichthärtend und niedrigviskos sind, so dass sie mit Hilfe von stereolithographischen Verfahren verarbeitbar sind, und die gehärteten Zusammensetzungen eine hohe Transparenz und eine geringe Eigenfarbe sowie eine hohe Bruchzähigkeit, insbesondere eine hohe Randfaserdehnung, bei gleichzeitig hoher Biegefestigkeit und hohem Biegemodul aufweisen. Die erfindungsgemäßen Zusammensetzungen eignen sich somit hervorragend als Dentalwerkstoff, insbesondere zur Herstellung von orthodontischen Vorrichtungen wie Alignern, Aligner-Attachments und Positionern.

Die erfindungsgemäßen Zusammensetzungen enthalten als radikalisch polymerisierbare Verbindung vorzugsweise zusätzlich zu dem Oligomer auch ein ein polyfunktionelles Monomer, das radikalisch polymerisierbar ist. Unter polyfunktionellen Monomeren werden Verbindungen mit zwei oder mehr, vorzugsweise 2 bis 6, wie z.B. 2 bis 3 oder 2 bis 4 radikalisch polymerisierbaren Gruppen verstanden. Bevorzugt weist das polyfunktionelle Monomer als radikalisch

polymerisierbare Gruppen (Meth)acrylate und/oder (Meth)acrylamide auf.

Besonders geeignete polyfunktionelle Monomere sind (Meth)acrylsäurederivate ausgewählt aus der Gruppe bestehend aus Bisphenol-A-di(meth)acrylat, Bis-G(M)A (ein Additionsprodukt aus (Meth)acrylsäure und Bisphenol-A-diglycidylether), ethoxy- oder propoxyliertem Bisphenol-A-di(meth)acrylat, wie z.B. Bisphenol-A-di(meth)acrylat mit 3 (SR-348c = Methacrylat; SR-349 = Acrylat, Fa. Sartomer) oder 2 Ethoxygruppen (SR-348L = Methacrylat, Fa. Sartomer), 2,2-Bis[4-(2-(meth)acryloxypropoxy)phenyl]propan, UD(M)A (ein Additionsprodukt aus 2-Hydroxyethyl(meth)acrylat und 2,2,4- oder 2,4,4-Trimethylhexamethylen-1,6-diisocyanat), Di-, Tri-, Tetra-, Penta-, Hexa- oder Hepta-ethylenglycoldi(meth)acrylat, Di-, Tri-, Tetra-, Penta-, Hexa- oder Hepta-propylenglycoldi-(meth)acrylat, Trimethylolpropantri(meth)acrylat, ethoxyliertem oder propopoxyliertem Trimethylolpropantri-(meth)acrylat, z.B. 3-fach propoxyliertem Trimethylolpropantriacrylat (Sartomer SR-492) und Tripropylenglycoldiacrylat, Pentaerythrittetra(meth)acrylat, sowie Glycerindi- und -tri(meth)acrylat, 1,4-Butandioldi(meth)acrylat, 1,10-Decan-dioldi(meth)acrylat ($D_3MA$), 1,12-Dodecandioldi(meth)acrylat, Polyether-(meth)acrylaten, Polyester-(meth)acrylaten, Epoxy-(meth)acrylaten, Urethan-(meth)acrylaten, Tricyclodecan-dimethanoldi(meth)acrylat, N,N-Dimethacrylamid, vernetzenden Pyrrolidonen wie 1,6-Bis-(3-vinyl-2-pyrrolidonyl)-hexan, Bisacrylamiden wie Methylen- oder Ethylenbisacrylamid und Bis(meth)acrylamiden wie N,N'-Diethyl-1,3-bis-(acrylamido)-propan, 1,3-Bis(methacrylamido)-propan, 1,4-Bis-(acrylamido)-butan oder 1,4-Bis(acryloyl)-piperazin.

Besonders bevorzugt sind di- und trifunktionelle Acrylate und Methacrylate mit einem Molekulargewicht von <1000 g/mol, wie z.B. Glycerin-1,3-dimethacrylat-2-acetat, aliphatische Urethan-diacrylate, Phthalsäure-HEA-Ester (Photomer 4173), Pyromellitsäuredi-di-HEA-Ester (HEA = 2-Hydroxyethylacrylat), Bis-G(M)A (ein Additionsprodukt aus (Meth)acrylsäure und Bisphenol-A-diglycidylether), 2,2-Bis[4-(2-(meth)acryloxy-propoxy)phenyl]propan, UD(M)A, Triethylenglycoldi(meth)acrylat (TEGD(M)A), 2-Phenoxethyl(meth)acrylat (Acrylat SR339C Fa Sartomer/ Arkema), Tricyclodecandimethanoldimethacrylat (CAS 43048-08-4) und FLKP-Vernetzer, d.h. das Umsetzungsprodukt aus 1,3-Phenylenbis(propan-2,2-diylcarbamoyloxyethan-2,1-diyl)-bis(2-methylacrylat) (CAS 178884-91-1), 2-{[(2-{-[2-Methacryloyloxy)ethoxy]carbonyl}amino)propan-2-yl]phenyl}propan-2-yl)carbamoyl]oxy}propylmethacrylat (CAS 1219495-43-8) und 1,3-Phenylenbis(propan-2,2-moyloxypropan-2,1-diyl)bis(2-methylacrylat) (CAS 138393-21-2). Diese Monomere zeichnen sich durch eine hohe Reaktivität, einen hohen Doppelbindungsumsatz, gute mechanische Eigenschaften, einen geringen Polymerisationsschrumpf und eine relativ niedrige Viskosität aus. Ganz besonders bevorzugt sind solche Zusammensetzungen, die als polyfunktionelles Monomer UDMA, TEGDMA, Bis-GMA, 2-Phenoxyethylacrylat (SR339C, Fa. Sartomer/Arkema), Glycerin-1,3-dimethacrylat-2-acetat oder eine Mischung davon, insbesondere eine Mischung von UDMA, TEGDMA und 2-Phenoxyethylacrylat oder vorzugsweise eine Mischung von UDMA, TEGDMA und Bis-GMA oder eine Mischung von UDMA und TEGDMA oder eine Mischung von UDMA, TEGDMA, Bis-GMA und Glycerin-1,3-dimethacrylat-2-acetat enthalten.

Die Verwendung von polyfunktionellen Monomeren, welche polare Gruppen aufwiesen, wie z.B. OH-Gruppen, Amidgruppen, Urethangruppen und/oder Harnstoffgruppen, haben sich als besonders vorteilhaft herausgestellt. Es wird angenommen, dass derartige polare Gruppen zur Ausbildung von intermolekularen Wasserstoffbrückenbindungen führen können, wodurch die Bruchzähigkeit der gehärteten Zusammensetzung nicht zu stark herabgesetzt wird.

Das radikalisch polymerisierbare polyfunktionelle Monomer oder eine Mischung davon liegt, bezogen auf das Gesamtgewicht der erfindungsgemäßen Zusammensetzung, vorzugsweise in einer Menge von 30 bis 65 Gew.-%, insbesondere 30 bis 60 Gew.-%, wie z.B. 35 bis 50 Gew.-%, besonders bevorzugt 38 bis 45 Gew.-%, insbesondere 38 bis 41 Gew.-% vor.

Die erfindungsgemäße Zusammensetzung kann ferner neben dem radikalisch polymerisierbaren Oligomer und dem radikalisch polymerisierbaren, polyfunktionellem Monomer optional ein oder mehrere monofunktionelle Monomere enthalten. Besonders geeignete monofunktionelle Monomere sind monofunktionelle (Meth)acrylate, wie z.B. Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E) und Dicyclopentanylmethacrylat (CAS Nr. 34759-34-7) . Besonders bevorzugt enthält die erfindungsgemäße Zusammensetzung Dicyclopentanylmethacrylat und/oder p-Cumylphenoxyethylenglycolmethacrylat (CMP-1E) als monofunktionelles Monomer.

Vorzugsweise liegt das monofunktionelle Monomer, bezogen auf das Gesamtgewicht der erfindungsgemäßen Zusammensetzung, in einer Menge von 0 bis 15 Gew.-%, besonders bevorzugt 0 bis 10 Gew.-%, insbesondere 5 bis 10 Gew.-% oder 7 bis 9 Gew.-% vor.

Bestimmte Eigenschaften der Werkstoffe vor und nach der Härtung lassen sich durch eine gezielte Kombinationen der Monomeren beeinflussen. Bei Mischungen aus monofunktionellen und difunktionellen Monomeren nimmt beispielsweise die Viskosität und Reaktivität mit dem Gehalt an monofunktionellen Monomeren ab. Mischungen aus difunktionellen und trifunktionellen Monomeren weisen eine höhere Reaktivität auf, wobei die Reaktivität mit dem Gehalt an trifunktionellen Monomeren zunimmt. Der Gehalt an trifunktionellen Monomeren bewirkt jedoch auch eine höhere Sprödigkeit der gehärteten Zusammensetzung. Reaktivität und Viskosität der ungehärteten Zusammensetzung sowie der Polymerisationsschrumpf werden außerdem durch die Molmasse der Monomeren bestimmt, wobei mit zunehmender Molmasse der Polymerisationsschrumpf abnimmt, während die Viskosität ansteigt.

Für Kombinationen von Oligomeren und polyfunktionellen Monomeren bzw. Kombinationen von Oligomeren, polyfunktionellen Monomeren und monofunktionellen Monomeren sind hingegen keine allgemeinen Gesetzmäßigkeiten bekannt. Vielmehr wurde von den vorliegenden Erfindern überraschenderweise gefunden, dass die Verwendung der speziellen, oben beschrieben Oligomere, insbesondere Oligomer (a1), in Kombination mit polyfunktionellen Monomeren, insbesondere in Kombination mit einer Mischung von UDMA, TEGDMA und Bis-GMA als polyfunktionelles Monomer und ggf. Dicyclopentanylmethacrylat als monofunktionelles Monomer, zu Materialien mit besonders günstigen Eigenschaften führt, nämlich zu Zusammensetzungen, die mit einfachen stereolithographischen Verfahren verarbeitbar sind, nach der Härtung eine hohe Bruchzähigkeit, insbesondere eine hohe Randfaserdehnung, bei gleichzeitig hoher Biegefestigkeit und hohem Biegemodul aufweisen und sich zudem durch eine geringe Eigenfarbe und eine hohe Transparenz auszeichnen.

Geeignete Photoinitiatoren (b) zur Initiierung der radikalischen Photopolymerisation sind Benzophenon, Benzoin sowie deren Derivate oder $\alpha$-Diketone oder deren Derivate, wie 9,10-Phenanthrenchinon, 1-Phenyl-propan-1,2-dion, Diacetyl oder 4,4'-Dichlorbenzil. Bevorzugt werden Campherchinon (CQ) und 2,2-Dimethoxy-2-phenyl-acetophenon und ganz besonders bevorzugt $\alpha$-Diketone in Kombination mit Aminen als Reduktionsmittel eingesetzt, wie z.B. 4-(Dimethylamino)-benzoesäureester (EDMAB), N,N-Dimethylaminoethylmethacrylat, N,N-Dimethyl-sym.-xylidin oder Triethanolamin. Weiter bevorzugt sind Diethylthioxanthen (DETX, CAS 82799-44-8) und Isopropylthioxanthon (ITX, CAS 75081-21-9), beide jeweils vorzugsweise in Kombination mit Ethyl-4-(dimethylamino)benzoat (EMBO, CAS Nr. 10287-53-3). Ebenso bevorzugt sind [1-/4-Phenylsulfanylbenzoyl)heptylidenamino]benzoat (Irgacure OXE 01), und [1[9-Ethyl-6-(2-methylbenzoyl)carbazol-3-yl]ethyl-idenamino]acetat (Irgacure OXE 02).

Besonders bevorzugte Photoinitiatoren sind weiterhin Norrish-Typ-I-Photoinitiatoren, vor allem Monoacyl- oder Bisacylphosphinoxide, wie z.B. Diphenyl-(2,4,6-trimethylbenzoyl)phenylphosphinoxid (CAS Nr. 75980-60-8), und insbesondere Monoacyltrialkyl- bzw. Diacyldialkyl- bzw. Tetraacylgermanium-Verbindungen, wie z.B. Benzoyltrimethylgermanium, Dibenzoyldiethylgermanium, Bis(4-methoxybenzoyl)di-ethylgermanium (MBDEGe), Tetrakis(4-ethoxybenzoyl)germanium oder Tetrakis(4-propoxybenzoyl)germanium, oder Acylzinn-Verbindungen, wie Monoacylstannane, Diacylstannane, Triacylstannane oder Tetraacylstannane, wie z.B. Benzoyltriphenylzinn. Weitere bevorzugte Acylgermanium-Verbindungen werden in der EP 3 150 641 A1 und weitere bevorzugte Acylzinn-Verbindungen werden in der EP 3 293 215 A1 beschrieben. Dabei lassen sich vorteilhaft auch Mischungen der verschiedenen Photoinitiatoren einsetzen, wie z.B. Bis(4-methoxybenzoyl)diethylgermanium in Kombination mit Campherchinon und 4-Dimethylaminobenzoesäureethylester.

Ganz besonders bevorzugt sind Campherchinon (CAS Nr. 10373-78-1) in Kombination mit Ethyl-4-(dimethylamino)benzoat (EMBO, CAS Nr. 10287-53-3) sowie Phenylbis(2,4,6-trimethylbenzoyl) phosphinoxid (Irgacure 819, CAS 162881-26-7), Diphenyl(2,4,6-trimethylbenzoyl)phenyl-phosphinoxid (TPO, CAS Nr. 75980-60-8), 2-Benzyl-2-(dimethylamino)-4'-morpholinobutyrophenon (Irgacure 369, CAS Nr. 119313-12-1), 1-Butanon-2-(dimethylamino)-2-(4-methylphenyl)methyl-1-4-(4-morpholinyl) phenyl (Irgacure 379, CAS-Nr. 119344-86-4) und ganz besonders Bis(4-methoxybenzoyl)diethylgermanium (MBDEGe; Ivocerin), Tetrakis(4-ethoxybenzoyl)germanium oder Tetrakis(4-propoxybenzoyl)germanium. Insbesondere sind die bevorzugten Photoinitiatoren solche, die entweder eine nur sehr geringe Eigenfarbe aufweisen oder bei Bestrahlung mit Licht, z.B. im stereolithographischen Druckprozess oder im Nachvergütungsprozess, ihre Farbe verlieren.

Die erfindungsgemäße Zusammensetzung enthält den Photoinitiator (b) vorzugsweise in einer Menge von 0,1 bis 3,0 Gew.-%, insbesondere 0,5 bis 2 Gew.-%, besonders bevorzugt 0,7 bis 1,5 Gew.-%, wie z.B. 0,8 bis 1,3 Gew.-%.

Neben den oben genannten Komponenten enthalten die erfindungsgemäßen Zusammensetzungen weiterhin mindestens einen UV-Absorber (c).

Der UV-Absorber dient dazu, bei der lichtinduzierten Härtung der erfindungsgemäßen Zusammensetzung die Eindringtiefe des Lichts und damit die Polymerisationstiefe zu verringern. Dies erweist sich insbesondere bei stereolithographischen Anwendungen als vorteilhaft, da bei der Stereolithographie lediglich dünne Schichten gehärtet werden sollen. Durch Verwendung eines UV-Absorbers kann somit bei stereolithographischen Verfahren die Präzision verbessert werden. Darüber hinaus können UV-Absorber als Farbmittel auch zu ästhetischen Zwecken zugesetzt werden.

Als UV-Absorber oder sog. Farbmittel sind organische Farbstoffe und Pigmente bevorzugt, insbesondere Azofarbstoffe, Carbonylfarbstoffe, Cyaninfarbstoffe, Azomethine und Methine, Phthalocyanine und Dioxazine. Besonders bevorzugt sind Farbstoffe, die in den Materialien löslich sind, insbesondere Azofarbstoffe. Als Farbmittel eignen sich außerdem anorganische und insbesondere organische Pigmente, die sich gut in den Materialien dispergieren lassen. Bevorzugt sind Azopigmente und Nichtazopimente. Beispielsweise sind UV-Absorber auf Basis von Benzotriazol, Benzophenon oder Triazine besonders geeignet. Bevorzugte UV-Absorber sind beispielsweise 2,2'-Methylenbis[6-(2H-benzotriazol-2-yl)-4-(1,1,3,3-tetramethylbutyl)phenol] (CAS-Nr. 103597-45-1), 2,2',4,4'-Tetrahydroxybenzophenon (CAS-Nr. 131-55-5), 2-tert-Butyl-6-(5-chloro-2H-benzotriazol-2-yl)-4-methylphenol (Bumetrizol; CAS-Nr. 3896-11-5), 2,2'-Benzol-1,4-diylbis(4h-3,1-benzoxazin-4-on) (CAS-Nr. 18600-59-4), 2-(4,6-Bis-(2,4-dimethylphenyl)-1,3,5-triazin-2-yl)-5-(octyloxy)-phenol (CAS-Nr. 2725-22-6), 2-(2-Hydroxy-5-methylphenyl)benzotriazol (CAS-Nr. 2440-22-4), 2-(2-Hydroxyphenyl)benzotriazol, 2-(2H-Benzotriazol-2-yl)-6-dodecyl-4-methylphenol (CAS-Nr. 23328-53-2), 2-(2'-Hydroxy-3', 5'-di-t-butylphenyl)-5-chlorobenzotriazole (CAS-Nr. 3864-99-1), 2,2'-Dihydroxy-4-methoxybenzophenon (CAS-Nr. 131-53-3) und 2,2'-Dihydroxy-4,4'-dimethoxybenzophenon (CAS-Nr. 131-54-4).

Ebenso geeignet sind sogenannte *Hindered Amine Light Stabilizers* wie Bis(1,2,2,6,6-pentamethyl-4-piperidyl)sebacat (CAS-Nr. 41556-26-7), Methyl-1,2,2,6,6-pentamethyl-4-piperidylsebacat (CAS-Nr. 82919-37-7), Bis(1-octyloxy-2,2,6,6-tetramethyl-4-piperidyl)sebacat (CAS-Nr. 129757-67-1) und Bis(1,2, 2,6,6-pentamethyl-4-piperidyl)-[[3,5-bis(1,1-dimethylethyl)-4-hydroxyphenyl]methyl]butylmalonat (CAS-Nr. 63843-89-0). Beispiele für weitere geeignete sogenannte *Hindered Amine Light Stabilizers* umfassen Verbindungen mit der CAS-Nr. 52829-07-9, CAS-Nr. 124172-53-8, CAS-Nr. 65447-77-0, CAS-Nr. 167078-06-0, CAS-Nr. 106990-43-6, CAS-Nr. 70624-18-9, CAS-Nr. 82451-48-7, CAS-Nr. 136504-96-6 oder CAS-Nr. 191743-75-6.

Besonders bevorzugt enthält die erfindungsgemäße Zusammensetzung Bumetrizol als UV-Absorber. In einer alternativen bevorzugten Ausführungsform ist der UV-Absorber 2,2',4,4'-Tetrahydroxybenzophenon.

Vorzugsweise enthält die Zusammensetzung mindestens einen UV-Absorber, der ein Absorptionsmaximum aufweist, das der Wellenlänge des zur Härtung eingesetzten Lichts entspricht. Sehr vorteilhaft sind UV-Absorber mit einem Absorptionsmaximum unterhalb von 400 nm oder im Bereich von 350 bis 550 nm, vorzugsweise 380 bis 480 nm.

Des Weiteren enthält die erfindungsgemäße Zusammensetzung neben einem UV-Absorber (c) ferner einen optischen Aufheller (d). Vorzugsweise ist der optische Aufheller (d) eine fluoreszierende Verbindung. Insbesondere absorbiert der optische Aufheller (d) vorzugsweise Licht im UV-Bereich, d.h. unterhalb von 400 nm. Dadurch kann die Eindringtiefe des Lichts und damit die Polymerisationstiefe verringert und die Präzision bei stereolithographischen Anwendungen erhöht werden. Außerdem ist der optische Aufheller vorzugsweise in der Lage, das im UV-Bereich aufgenommene Licht als Licht mit einer Wellenlänge von vorzugsweise 400 bis 450 nm abzugeben. Der optische Aufheller kann somit ferner zu einer Erhöhung der Reaktivität der Zusammensetzung führen, da der Aufheller das aufgenommene Licht aufgrund seiner Fluoreszenz wieder als Blaulicht abstrahlt und damit zusätzliche Lichtleistung zur Photoinitiierung bereitstellt.

Vorzugsweise ist der optische Aufheller (d) ausgewählt aus der Gruppe bestehend aus 2,5-Bis(5-tert-butylbenzoxazol-2-yl)thiophen (CAS-Nr. 7128-64-5) Fluoreszenzmitteln in Form von Terephthalsäurederivaten, wie z.B. Terephthalsäurederivaten, die z.B. unter der Bezeichnung Lumilux blau LZ von der Firma Honeywell Specialty Chemicals Seelze, Deutschland, erhältlich sind, oder Diethyl-2,5-dihydroxyterephthalat (CAS-Nr. 5870-38-2) .

Vorzugsweise liegt der UV-Absorber (c), bezogen auf das Gesamtgewicht der erfindungsgemäßen Zusammensetzung, in einer Menge von 0 bis 2,0 Gew.-%, besonders bevorzugt 0,0001 bis 0,5 Gew.-% vor. Im Fall von Bumetrizol als UV-Absorber beträgt dessen Menge vorzugsweise 0,01 bis 0,2 Gew.-%, besonders bevorzugt 0,02 bis 0,15 Gew.-%. Im Fall von 2,2',4,4'-Tetrahydroxybenzophenon als UV-Absorber ist die Menge vorzugsweise geringer und beträgt insbesondere 0,01 bis 0,07 Gew.-%. Bei Zusammensetzungen, die einen oder mehrere Füllstoffe enthalten, ist die Menge an UV-Absorber (c) geringer als bei ungefüllten Zusammensetzungen.

Des Weiteren ist es bevorzugt, dass der optische Aufheller (d), bezogen auf das Gesamtgewicht der erfindungsgemäßen Zusammensetzung, in einer Menge von 0,001 bis 0,1 Gew.-%, vorzugsweise 0,002 bis 0,05 Gew.-%, besonders bevorzugt 0,005 bis 0,02 Gew.-%, vorliegt.

Außerdem ist es bevorzugt, dass das Gewichtsverhältnis von UV-Absorber (c) zu optischem Aufheller (d) 2:1 bis 50:1, vorzugsweise 2:1 bis 30:1, besonders bevorzugt 2:1 bis 5:1 oder 10:1 bis 25:1, beträgt.

Es wurde gefunden, dass derartige Kombinationen von UV-Absorber (c) und optischem Aufheller (d) nach der Aushärtung der erfindungsgemäßen Zusammensetzung zu besonders transparenten Materialien ohne visuell wahrnehmbare Eigenfarbe führt. Besonders bevorzugt ist die erfindungsgemäße Zusammensetzung nach der Härtung durch eine Transparenz von 80% oder mehr, insbesondere 90% oder mehr gekennzeichnet, gemessen als Hellbezugswert mittels Spektrophotometer Minolta CM-5 im Transmissionsmodus gegen eine Lichtquelle D65 als Parameter Y im Yxy Farbraum an 2 mm dicken hochglanzpolierten Prüfkörpern.

Besonders ideale Ergebnisse mit besonders hoher Transparenz und besonders geringer Eigenfarbe lassen sich erzielen, wenn die erfindungsgemäße Zusammensetzung als UV-Absorber (c) 2,2',4,4'-Tetrahydroxybenzophenon oder Bumetrizol und als optischen Aufheller (d) 2,5-Bis(5-tert-butyl-benzoxazol-2-yl)thiophen enthält. Insbesondere enthält die erfindungsgemäße Zusammensetzung 2,2',4,4'-Tetrahydroxybenzophenon und 2,5-Bis(5-tert-butyl-benzoxazol-2-yl)thiophen in einem Gewichtsverhältnis von 2:1 bis 10:1, vorzugsweise 2: bis 5:1, oder Bumetrizol und 2,5-Bis(5-tert-butyl-benzoxazol-2-yl)thiophen in einem Gewichtsverhältnis von 5:1 bis 30:1, vorzugsweise 10:1 bis 20:1.

Die erfindungsgemäßen Zusammensetzungen können außerdem partikulären Füllstoff (e) enthalten. Generell können als Füllstoffe anorganische und/oder organische Partikel eingesetzt werden. Der oder die Füllstoffe haben vorzugsweise eine Partikelgröße von weniger als 25 $\mu$m, bevorzugt weniger als 10 $\mu$m und besonders bevorzugt weniger als 5 $\mu$m. Bei allen Partikelgrößen hierin handelt es sich, wenn nicht anders angegeben, um D50-Werte, d.h. 50 Vol.-% der Partikel haben einen Durchmesser, der kleiner als der angegebene Wert ist. Füllstoffe dienen primär dazu, die Viskosität der härtbaren Zusammensetzung sowie die mechanischen und/oder optischen Eigenschaften der gehärteten Werkstoffe einzustellen. Des Weiteren ist es bevorzugt, dass die maximale Partikelgröße kleiner als die Dicke der stereolithographisch erzeugten Schichten ist. Bevorzugt sind Partikel mit einer maximalen Größe von 25 $\mu$m, vorzugs-

weise maximal 15 µm.

Die Oberfläche der Füllstoffe kann modifiziert werden, beispielsweise um die Dispergierbarkeit der Füllstoffe in der organischen Matrix der erfindungsgemäßen Zusammensetzung zu verbessern. Zur Oberflächenmodifizierung werden vorzugsweise solche Verbindungen eingesetzt, die chemisch, d.h. durch ionische oder kovalente Bindungen an die Oberfläche der Füllstoffe gebunden werden. Bevorzugt sind Verbindungen, die entweder Säuregruppen, vorzugsweise Carbonsäure-, Phosphonsäure-, Hydrogenphosphatgruppen oder saure Phosphorsäureestergruppen, oder Silylgruppen, vorzugsweise Alkoxysilylgruppen enthalten. Die Partikeloberfläche kann teilweise oder vorzugsweise vollständig mit dem Modifizierungsmittel bedeckt sein. Bei den erfindungsgemäß verwendeten Modifizierungsmitteln handelt es sich um monomere Verbindungen. Als Oberflächenmodifizierungsmittel eigen sich besonders lineare Carbonsäuren, wie z.B. Ameisensäure, Essigsäure, Propionsäure, Octansäure, Isobuttersäure, Isovaleriansäure, Pivalinsäure, oder Phosphonsäuren, z.B. wie Methyl-, Ethyl-, Propyl-, Butyl- Hexyl, Octyl- oder Phenylphosphonsäure. Als silylgruppenhaltige Verbindungen sind Silane wie Propyltrimethoxysilan, Phenyltrimethoxysilan, Hexyltrimethoxysilan, Octyltrimethoxysilan, Trimethylchlorsilan, Trimethylbromsilan, Trimethylmethoxysilan und Hexamethyldisilazan bevorzugt. Ganz besonders bevorzugte Oberflächenmodifizierungsmittel sind saure Phosphorsäureester, wie z.B. Dimethyl-, Diethyl-, Dipropyl-, Dibutyl-, Dipentyl-, Dihexyl-, Dioctyl- oder Di(2-ethylhexyl)phosphat. Die Oberflächenmodifizierungsmittel können auch radikalisch polymerisierbare Gruppen, beispielsweise (Meth)acrylatgruppen, aufweisen, die mit der Komponente (a) reagieren und so in das Polymernetzwerk eingebunden werden.

Bevorzugte Füllstoffe sind partikuläres Wachse, insbesondere Carnaubawachs, vorzugsweise mit eine Partikelgröße von 1 bis 10 µm, vernetzte Polymethylmethacrylat (PMMA)-Partikel, vorzugsweise mit einer Partikelgröße von 500 nm bis 10 µm, sowie Polyamid-12 Partikel, vorzugsweise mit einer Partikelgröße von 5 bis 10 µm. Ganz besonders bevorzugte Füllstoffe sind Glasfüllstoffe, insbesondere Glasfüllstoffe, die vor einer etwaigen Oberflächenmodifizierung eine Partikelgröße von 0,2 bis 2,0 µm, vorzugsweise 0,5 bis 1,5 µm, aufweisen. Ebenfalls besonders bevorzugt enthält die Zusammensetzung einen sog. Präpolymerfüllstoff oder Isofüllstoff, d.h. ein gemahlenes Kompositmaterial, das vorzugsweise eine breite Partikelgrößenverteilung z. B. mit Partikelgrößen von 0,05 bis 20 µm, insbesondere etwa 0,1 bis etwa 10 µm, aufweist. In einer Ausführungsform weist der Präpolymerfüllstoff oder Isofüllstoff vorzugseise eine bimodale Partikelgrößenverteilung auf, wobei vorzugsweise mehr als 50%, insbesondere mehr als 70% und besonders bevorzugt mehr als 80% der Füllstoffpartikel eine Partikelgröße von 1 bis 15 µm aufweisen, gemessen mittels Laserdiffraktion mit CILAS 1064 Korngrößenbestimmungsgerät in Wasser mit Dispergiermittel Teepol (Teepol L von BDH Laboratory SUPPLIES; poole BH15 1 TD, England) unter Ultraschall-Dispergierung. Vorzugsweise ist der Präpolymerfüllstoff oder Isofüllstoff oberflächenmodifiziert, insbesondere silanisiert. Im Falle von oberflächenmodifizierten Präpolymer- oder Isofüllstoffen beziehen sich die oben genannten Partikelgrößen auf die Partikelgrößen vor der Oberflächenmodifizierung, d.h. der Füllstoffe vor z.B. Silanisierung.

In einer bevorzugten Ausführungsform enthält die erfindungsgemäße Zusammensetzung eine Mischung von zwei oder mehreren Füllstoffen, insbesondere von zwei oder mehreren Füllstoffen mit verschiedenen Partikelgrößen. Es wurde gefunden, dass durch die Verwendung solcher Füllstoffmischungen die Viskosität der Zusammensetzung nicht übermäßig erhöht wird und die Zusammensetzungen daher weiterhin mit generativen Verfahren, wie z.B. mit Hilfe von Stereolithographie, verarbeitbar sind. Besonders bevorzugt ist eine Mischung eines silanisierten Glasfüllers (i) mit einer Partikelgröße von etwa 0,7 µm (vor der Silanisierung), eines silanisierten Glasfüllers (ii) mit einer Partikelgröße von etwa 1,0 µm (vor der Silanisierung) und eines Präpolymerfüllstoffs (iii) mit einer breiten Partikelgrößenverteilung von etwa 0,1 bis etwa 10 µm. Des Weiteren ist es ganz besonders bevorzugt, das die erfindungsgemäße Zusammensetzung, bezogen auf ihr Gesamtgewicht, 2,0 bis 4,0 Gew.-%, insbesondere 2,5 bis 3,5 Gew.-% von Glasfüllstoff (i), 0,5 bis 2,0 Gew.-%, insbesondere 1,0 bis 1,6 Gew.-%, von Glasfüllstoff (ii) und 4,0 bis 6,0 Gew.-%, insbesondere 4,5 bis 5,5 Gew.-% von Präpolymerfüllstoff (iii) enthält.

In einer alternativen bevorzugten Ausführungsform ist die erfindungsgemäße Zusammensetzung frei von Füllstoffen (e).

Im Allgemeinen enthält die erfindungsgemäße Zusammensetzung vorzugsweise 0 bis 15 Vol.-% an Füllstoff(en) (e). Dies entspricht vorzugsweise einer Menge von 0 bis 40 Gew.-%, insbesondere 0 bis 20 Gew.-% und besonders bevorzugt 0 Gew.-% oder 6,0 bis 20,0 Gew.-%, wie z.B. 8,0 bis 15,0 Gew.-% oder 8,0 bis 10,0 Gew.-%, an Füllstoff (e), bezogen auf das Gesamtgewicht der erfindungsgemäßen Zusammensetzung.

Für den Fall, dass die die erfindungsgemäße Zusammensetzung einen oder mehrere Füllstoffe (e) umfasst, enthält die Zusammensetzung vorzugsweise ferner auch ein Thixotropieadditiv (f). Dies Additiv (f) stellt ein Verdickungsmittel dar und dient dazu, ein Sedimentieren der Füllstoffe in der Zusammensetzung zu verhindern. Um die Viskosität der Zusammensetzung nicht zu stark zu erhöhen, enthält die Zusammensetzung vorzugsweise nur geringe Mengen an Thixotropieadditiv (f), wie z.B. 0 bis 3,0 Gew.-%, insbesondere 0 bis 2,0 Gew.-%, besonders bevorzugt 0,1 bis 2,0 Gew.-% oder 0,5 bis 2,0 Gew.-%, bezogen auf das Gesamtgewicht der Zusammensetzung. Bevorzugte Thixotropieadditive (f) sind insbesondere ausgewählt aus der Gruppe bestehend aus $SiO_2$, hochdispersem $SiO_2$, d.h. $SiO_2$ mit geringer Primärteilchengröße und hoher Oberfläche, Polymeren, Blockcopolymeren und Schichtsilicaten.

Neben den oben genannten Komponenten können die erfindungsgemäßen Zusammensetzungen ein oder

mehrere weitere Additive enthalten.

Beispielsweise können die Zusammensetzungen einen oder mehrere Polymerisationsinhibitoren enthalten. Der oder die Polymerisationsinhibitoren dienen als Stabilisator zur Verhinderung einer spontanen Polyreaktion. Die Inhibitoren bzw. Stabilisatoren verbessern die Lagerstabilität der Zusammensetzungen und verhindern darüber hinaus eine unkontrollierte Polyreaktion in der stereolithographischen Wanne. Die Inhibitoren werden vorzugsweise in einer solchen Menge zugesetzt, dass die Zusammensetzungen über einen Zeitraum von ca. 2-3 Jahre lagerstabil sind. Besonders bevorzugt werden die Inhibitoren in einer Menge von 0,001 bis 1,0 Gew.-%, ganz besonders bevorzugt 0,001 bis 0,20 Gew.-% eingesetzt, jeweils bezogen auf die Gesamtmasse der Zusammensetzung.

Bevorzugt sind sogenannte aerobe Inhibitoren Phenole, wie Hydrochinonmonomethylether (MEHQ) oder 2,6-Di-tert.-butyl-4-methyl-phenol (BHT), eingesetzt, die nur in Gegenwart von Sauerstoff effektiv wirksam sind und vorzugsweise in einem Konzentrationsbereich von 100-2000 ppmw verwendet werden. Geeignete anaerobe Inhibitoren sind Phenothiazin, 2,2,6,6-Tetramethyl-piperindin-1-oxyl-Radikal (TEMPO), Iod und Kupfer-(I)-iodid. Diese wirken schon in geringen Konzentrationen von vorzugsweise 10-200 ppmw auch bei Abwesenheit von Sauerstoff. Eine Polymerisation findet erst dann statt, wenn diese Additive verbraucht sind. Dabei ist es von Vorteil, eine Mischung von aeroben und anaeroben Inhibitoren einzusetzen.

Aerobe Inhibitoren werden vorzugsweise in einer Menge von 0,001 bis 0,50 Gew.-% und anaerobe Inhibitoren in einer Menge von 0,001 bis 0,02 Gew.-% eingesetzt, jeweils bezogen auf die Gesamtmasse der Zusammensetzung. Bevorzugte Mischungen enthalten 0,005-0,10 Gew.-% an aeroben Inhibitoren und 0,001-bis 0,02 Gew.-% an anaeroben Inhibitoren, ebenfalls bezogen auf die Gesamtmasse der Zusammensetzung.

Mithin weisen die erfindungsgemäßen Zusammensetzungen vorzugsweise folgende Zusammensetzung auf:

(a') 25 bis 60 Gew.-% , insbesondere 30 bis 55 Gew.-%, wie z.B. 32 bis 50 Gew.-%, besonders bevorzugt 38 bis 42 Gew.-% Oligomer, vorzugsweise Oligomer (a1),
(a") 30 bis 65 Gew.-%, insbesondere 30 bis 60 Gew.-%, bevorzugter 35 bis 50 Gew.-%, besonders bevorzugt 38 bis 45 Gew.-%, insbesondere 38 bis 41 Gew.-% polyfunktionelles Monomer,
(b) 0,1 bis 3,0 Gew.-%, insbesondere 0,5 bis 2 Gew.-%, besonders bevorzugt 0,7 bis 1,5 Gew.-%, insbesondere 0,8 bis 1,3 Gew.-% Photoinitiator,
(a''') 0 bis 15 Gew.-%, besonders bevorzugt 0 bis 10 Gew.-%, insbesondere 5 bis 10 Gew.-% oder 7 bis 9 Gew.-% monofunktionelles Monomer,
(c) 0,0001 bis 2,0 Gew.-%, besonders bevorzugt 0,0001 bis 0,5 Gew.-% UV-Absorber,
(d) 0,001 bis 0,1 Gew.-%, besonders bevorzugt 0,002 bis 0,05 Gew.-% optischer Aufheller,
(e) 0 bis 40 Gew.-%, insbesondere 0 bis 20 Gew.-%, besonders bevorzugt 0 Gew.-% oder 6,0 bis 20,0 Gew.-%, insbesondere 8,0 bis 15,0 Gew.-% oder 8,0 bis 10,0 Gew.-% Füllstoff und
(f) 0 bis 3,0 Gew.-%, insbesondere 0 bis 2,0 Gew.-%, besonders bevorzugt 0,1 bis 2,0 Gew.-% oder 0,5 bis 2,0 Gew.-% Thixotropieadditiv,

jeweils bezogen auf das Gesamtgewicht der Zusammensetzung.

Eine ganz besonders bevorzugte Zusammensetzung enthält:

(a') 40 bis 60 Gew.-% , insbesondere 45 bis 55 Gew.-%, wie z.B. 48 bis 52 Gew.-% Oligomer, vorzugsweise Oligomer (a1),
(a") 40 bis 60 Gew.-%, insbesondere 45 bis 55 Gew.-%, besonders bevorzugt 46 bis 52 Gew.-%, polyfunktionelles Monomer, wobei das polyfunktionelle Monomer vorzugsweise eine Mischung von UDMA, TEGDMA und Bis-GMA ist,
(b) 0,5 bis 2 Gew.-%, besonders bevorzugt 0,7 bis 1,5 Gew.-%, insbesondere 0,8 bis 1,3 Gew.-% Photoinitiator,
(a''') 0 bis 10 Gew.-%, besonders bevorzugt 0 bis 5 Gew.-%, insbesondere 0 Gew.-% monofunktionelles Monomer,
(c) 0,0001 bis 2,0 Gew.-%, besonders bevorzugt 0,01 bis 0,5 Gew.-%, besonders bevorzugt 0,05 bis 0,3 Gew.-% UV-Absorber,
(d) 0,001 bis 0,1 Gew.-%, besonders bevorzugt 0,002 bis 0,05 Gew.-% optischer Aufheller,(e) 0 bis 40 Gew.-%, insbesondere 0 bis 20 Gew.-%, besonders bevorzugt 0 Gew.-% Füllstoff und
(f) 0 bis 3,0 Gew.-%, insbesondere 0 bis 2,0 Gew.-%, besonders bevorzugt 0 Gew.-% Thixotropieadditiv,

jeweils bezogen auf das Gesamtgewicht der Zusammensetzung, wobei die Mischung von UDMA, TEGDMA und Bis-GMA vorzugsweise, bezogen auf das Gesamtgewicht der Zusammensetzung,

(a"1) 10 bis 20 Gew.-%, besonders bevorzugt 12 bis 18 Gew.-% UDMA,
(a"2) 20 bis 35 Gew.-%, besonders bevorzugt 25 bis 33 Gew.-% TEGDMA und
(a"3) 3 bis 20 Gew.-%, besonders bevorzugt 3 bis 10 Gew.-% Bis-GMA

enthält.

In einer weiteren ganz bevorzugten Ausführungsform enthält die Zusammensetzung:

(a’) 37 bis 55 Gew.-% , insbesondere 39 bis 47 Gew.-%, wie z.B. 43 bis 46 Gew.-% Oligomer, vorzugsweise Oligomer (a1),
(a") 39 bis 56 Gew.-%, insbesondere 42 bis 47 Gew.-% polyfunktionelles Monomer, wobei das polyfunktionelle Monomer vorzugsweise eine Mischung von UDMA, TEGDMA und Bis-GMA ist,
(b) 0,5 bis 2 Gew.-%, besonders bevorzugt 0,7 bis 1,5 Gew.-%, insbesondere 0,8 bis 1,3 Gew.-% Photoinitiator,
(a’’’) 0 bis 15 Gew.-%, besonders bevorzugt 7 bis 14 Gew.-%, insbesondere 8 bis 12 Gew.-% monofunktionelles Monomer, wobei das monofunktionelle Monomer (a) vorzugsweise Dicyclopentanylmethacrylat und/oder p-Cumylphenoxyethylenglycolmethacrylat und insbesondere Dicyclopentanylmethacrylat enthält,
(c) 0,0001 bis 2,0 Gew.-%, besonders bevorzugt 0,001 bis 0,5 Gew.-%, besonders bevorzugt 0,01 bis 0,1 Gew.-% UV-Absorber,
(d) 0,001 bis 0,1 Gew.-%, besonders bevorzugt 0,002 bis 0,05 Gew.-% optischer Aufheller,
(e) 0 bis 40 Gew.-%, insbesondere 0 bis 20 Gew.-%, besonders bevorzugt 0 Gew.-% Füllstoff und
(f) 0 bis 3,0 Gew.-%, insbesondere 0 bis 2,0 Gew.-%, besonders bevorzugt 0 Gew.-% Thixotropieadditiv,

jeweils bezogen auf das Gesamtgewicht der Zusammensetzung, wobei die Mischung von UDMA, TEGDMA und bisGMA vorzugsweise, bezogen auf das Gesamtgewicht der Zusammensetzung,

(a"1) 10 bis 20 Gew.-%, besonders bevorzugt 12 bis 16 Gew.-% UDMA,
(a"2) 20 bis 35 Gew.-%, besonders bevorzugt 21 bis 29 Gew.-% TEGDMA und
(a’’3) 3 bis 20 Gew.-%, besonders bevorzugt 3 bis 10 Gew.-% Bis-GMA

enthält.

In einer weiteren ganz bevorzugten Ausführungsform enthält die Zusammensetzung:

(a’) 37 bis 55 Gew.-% , insbesondere 38 bis 44 Gew.-%, wie z.B. 39 bis 42 Gew.-% Oligomer, vorzugsweise Oligomer (a1),
(a") 35 bis 50 Gew.-%, insbesondere 37 bis 42 Gew.-% polyfunktionelles Monomer, wobei das polyfunktionelle Monomer vorzugsweise eine Mischung von UDMA, TEGDMA und Bis-GMA ist,
(b) 0,5 bis 2 Gew.-%, besonders bevorzugt 0,7 bis 1,5 Gew.-%, insbesondere 0,8 bis 1,3 Gew.-% Photoinitiator,
(a’’’) 0 bis 15 Gew.-%, besonders bevorzugt 7 bis 14 Gew.-%, insbesondere 8 bis 12 Gew.-% monofunktionelles Monomer, wobei das monofunktionelle Monomer (a) vorzugsweise Dicyclopentanylmethacrylat und/oder p-Cumylphenoxyethylenglycolmethacrylat und insbesondere Dicyclopentanylmethacrylat enthält,
(c) 0 bis 2,0 Gew.-%, besonders bevorzugt 0,001 bis 0,5 Gew.-%, besonders bevorzugt 0,01 bis 0,1 Gew.-% UV-Absorber,
(d) 0,001 bis 0,1 Gew.-%, besonders bevorzugt 0,002 bis 0,05 Gew.-% optischer Aufheller,
(e) 0 bis 40 Gew.-%, insbesondere 0 bis 20 Gew.-%, besonders bevorzugt 7 bis 15 Gew.-% oder 8 bis 12 Gew.-% Füllstoff, wobei der Füllstoff vorzugsweise eine Mischung der oben beschriebenen Füllstoffe (i), (ii) und (iii) ist, und
(f) 0 bis 3,0 Gew.-%, insbesondere 0,1 bis 2,0 Gew.-%, besonders bevorzugt 0,5 bis 1,6 Gew.-% Thixotropieadditiv, wobei das Thixotropieadditiv vorzugsweise $SiO_2$ ist,

jeweils bezogen auf das Gesamtgewicht der Zusammensetzung, wobei die Mischung von UDMA, TEGDMA und Bis-GMA vorzugsweise, bezogen auf das Gesamtgewicht der Zusammensetzung,

(a"1) 10 bis 20 Gew.-%, besonders bevorzugt 10 bis 15 Gew.-% UDMA,
(a"2) 20 bis 35 Gew.-%, besonders bevorzugt 21 bis 26 Gew.-% TEGDMA und
(a"3) 3 bis 20 Gew.-%, besonders bevorzugt 3 bis 8 Gew.-% bisGMA

enthält.

Die rheologischen Eigenschaften der erfindungsgemäßen Zusammensetzungen werden an den gewünschten Anwendungszweck angepasst. Materialen zur stereolithographischen Verarbeitung werden vorzugsweise so eingestellt, dass ihre Viskosität im Bereich von 50 mPa·s bis 100 Pa·s, bevorzugt 100 mPa·s bis 10 Pa·s, besonders bevorzugt 100 mPa·s bis 5 Pa·s liegt. Die Viskosität wird bei der gewünschten Verarbeitungstemperatur der Materialiens mit einem Kegel-Platte-Viskosimeter bestimmt (Scherrate 100/s). Die Verarbeitungstemperatur liegt vorzugsweise im Bereich von 10 bis 70°C, besonders bevorzugt 20 bis 30°C. Besonders bevorzugt weist die erfindungsgemäße Zusammensetzung eine Viskosität < 10 Pa·s bei 25°C auf. Aufgrund der geringen Viskosität ist die erfindungsgemäße Zusammensetzung

dazu geeignet, mit Hilfe von generativen Fertigungsverfahren, wie z.B. 3D-Druck oder Stereolithographie, gehärtet zu werden.

Des Weiteren zeichnet sich die erfindungsgemäße Zusammensetzung dadurch aus, dass sie vor und insbesondere auch nach der Lichthärtung eine hohe Transparenz und eine geringe Eigenfarbe aufweist.

Des Weiteren zeichnet sich die erfindungsgemäße Zusammensetzung dadurch aus, dass die nach der Härtung erhaltenen Materialien eine hohe Bruchzähigkeit aufweisen. Werkstücke, die durch Härtung der erfindungsgemäßen Zusammensetzung erhalten werden, halten somit in hohem Maße Verformungen ohne Bruch stand. Vorzugsweise weist ein gehärtetes Werkstück eine Randfaserdehnung von mehr als 7 %, insbesondere eine Randfaserdehnung mehr als 10 %, besonders bevorzugt eine Randfaserdehnung von mehr als 11 %, wie z.B. eine Randfaserdehnung von 12 % auf. Die Randfaserdehnung wird in einem Dreipunktbiegeversuch gemäß ISO 4049 mit einer Stützweite von 20 mm bestimmt, wobei an der Unterseite des Prüfkörpers ein Längenaufnehmer angeordnet ist, um die exakte Durchbiegung an der Probenkörperunterseite zu bestimmen. Die Randfaserdehnung in Prozent ($\varepsilon_f$) wird mit der Formel $\varepsilon_f = (600 \cdot s1 \cdot h)/L^2$ berechnet, wobei s1 die Durchbiegung beim Bruch des Prüfkörpers, gemessen an der Prüfkörperunterseite, h die Höhe des Prüfkörpers und L die Stützweite ist. Besonders bevorzugt weist die erfindungsgemäße Zusammensetzung nach der Härtung eine derartige Randfaserdehnung auf, dass Prüfkörper bei einem Biegeversuch gemäß ISO 4049 mit einer Stützweite von 20 mm bei einer Durchbiegung von 4 mm nicht brechen.

Außerdem zeichnet sich die erfindungsgemäße Zusammensetzung dadurch aus, dass die nach der Härtung erhaltenen Materialien eine hohe Biegefestigkeit und ein hohes Biegemodul aufweisen. Werkstücke, die durch Härtung der erfindungsgemäßen Zusammensetzung erhalten werden, weisen somit eine hohe Steifigkeit auf und setzen einer Verformung einen hohen Widerstand entgegen. Vorzugsweise weist ein gehärtetes Werkstück eine Biegefestigkeit, bestimmt gemäß ISO 4049, von 40 bis 140 MPa, insbesondere eine Biegefestigkeit von 50 MPa oder mehr wie z.B. 50 bis 80 MPa, besonders bevorzugt eine Biegefestigkeit von mehr als 55 MPa auf. Ferner weist ein gehärtetes Werkstück vorzugsweise ein Biegemodul, bestimmt gemäß ISO 4049, von 800 bis 3800 MPa wie 1000 bis 2500 MPa, insbesondere ein Biegemodul von mehr als 1000 MPa, besonders bevorzugt ein Biegemodul von mehr als 1200 MPa oder mehr als 1300 oder mehr als 1500 oder mehr als 1800 MPa auf.

Des Weiteren zeichnet sich die erfindungsgemäße Zusammensetzung dadurch aus, dass die nach der Härtung erhaltenen Materialien eine hohe Bruchzähigkeit und eine hohe Bruchabeit aufweisen. Vorzugsweise weist ein Werkstück, das durch die Härtung der erfindungsgemäßen Zusammensetzung erhältlich ist, eine Bruchzähigkeit $K_{max}$ von 0,5 bis 1,8 $MPa \cdot m^{1/2}$, insbesondere von mindestens 0,6 $MPa \cdot m^{1/2}$, besonders bevorzugt mindestens 0,8 $MPa \cdot m^{1/2}$ oder mindestens 1,0 $MPa \cdot ^{1/2}$ oder mindestens 1,2 $MPa \cdot m^{1/2}$, und/oder eine Bruchabeit FW von 70 bis 600 $J/m^2$, wie z.B. 100 bis 450 $J/m^2$, insbesondere von mindestens 110 $J/m^2$, besonders bevorzugt mindestens 150 $J/m^2$ oder mindestens 180 $J/m^2$ oder mindestens 250 $J/m^2$, auf, wobei die Bruchzähigkeit $K_{max}$ und die Brucharbeit FW gemäß dem unten beschriebenen Testverfahren bestimmt werden.

Aufgrund der obigen Eigenschaften eignen sich erfindungsgemäße Zusammensetzungen hervorragend als Dentalwerkstoff und insbesondere zur Herstellung oder Reparatur von dentalen Werkstücken. Mithin betrifft die vorliegende Erfindung auch die Verwendung einer erfindungsgemäßen Zusammensetzung als Dentalwerkstoff und insbesondere zur Herstellung oder Reparatur von dentalen Werkstücken, wie z.B. Dentalrestaurationen, Prothesen, Prothesenwerkstoffen, künstlichen Zähnen, Inlays, Onlays, Kronen, Brücken, Bohrschablonen, Einprobekörpern oder orthodontischen Vorrichtungen. Besonders bevorzugt eignet sich die erfindungsgemäße Zusammensetzung zur Herstellung von orthodontischen Vorrichtungen, insbesondere Alignern, Positionern, Aligner-Attachments, Aligner-Attachments tragenden Positionern, Aufbissschienen (Splints), Transferschienen oder orthodontischen Schienen.

Mithin betrifft die vorliegende Erfindung auch ein Verfahren zur Herstellung von dentalen Werkstücken, insbesondere zur Herstellung von den oben genannten dentalen Werkstücken, bei dem eine erfindungsgemäße Zusammensetzung mit Hilfe von Licht gehärtet wird, um das dentale Werkstück zu ergeben. Weiterhin betrifft die Erfindung auch dentale Werkstücke, insbesondere die oben genannten Werkstücke, die durch ein derartiges Verfahren erhältlich sind.

Die Herstellung oder Reparatur von dentalen Werkstücken erfolgt insbesondere extraoral. Des Weiteren ist es bevorzugt, dass die Herstellung oder Reparatur von dentalen Werkstücken durch ein generatives Verfahren, insbesondere mit Hilfe von 3D-Druck oder einem Lithographie-basierten Verfahren, wie z.B. Stereolithographie, erfolgt.

Die Herstellung des erfindungsgemäßen dentalen Werkstücks erfolgt vorzugsweise durch ein stereolithographisches Verfahren. Hierzu wird durch die direkte oder indirekte Digitalisierung des zu restaurierenden Zahns bzw. der zu restaurierenden Zähne am Computer ein virtuelles Abbild der Zahnsituation erstellt, dann anhand dieses Abbilds am Computer ein Modell der Dentalrestauration konstruiert und dieses dann durch generative stereolithographische Fertigung hergestellt. Die Herstellung einer orthodontischen Vorrichtung mit Hilfe eines stereolithographischen Verfahrens wird entsprechend durchgeführt. Nach Erstellung eines virtuellen Modells der herzustellenden orthodontischen Vorrichtung wird die erfindungsgemäße Zusammensetzung durch selektive Lichteinstrahlung polymerisiert. Die Geometrie der orthodontischen Vorrichtung kann schichtweise aufgebaut werden, indem nacheinander eine Vielzahl von dünnen Schichten mit gewünschtem Querschnitt polymerisiert wird. Nach dem schichtweisen Aufbau der Geometrie schließt sich üblicherweise eine Reinigung des Werkstücks durch Behandlung mit einem geeigneten Lösungsmittel, wie z.B.

einem Alkohol, wie Ethanol oder Isopropanol, einem Keton, einem Ketal oder einem Ester, und eine Nachvergütung durch Bestrahlung des Werkstücks mit einer geeigneten Wellenlänge, wie z.B. einer Bestrahlung mit einer Intensität von 25 $mW/cm^2$ bei 405 nm und gleichzeitig 130 mW bei 406 nm für 15 min, an, bei der das Werkstück mit Licht geeigneter Wellenlänge bestrahlt wird, um den Restmonomergehalt weiter zu verringern und die mechanischen Eigenschaften zu verbessern.

Die Erfindung wird im Folgenden anhand von Ausführungsbeispielen näher erläutert.

**Ausführungsbeispiele**

Beispiele 1 bis 14

Die in den Tabellen 1 bis 2 aufgeführten Komponenten wurden in den angegebenen Mengen homogen miteinander gemischt. Dazu wurden in einem Rührwerk mit Dissolverscheibe mit Ausnahme der Glasfüller und Präpolymerfüller alle festen Komponenten in den Monomeren unter Rühren für etwa 60 min gelöst. Anschließend wurde das Oligomer hinzugegeben und es wurde bis zum Erreichen eines homogenen Gemisches gerührt (etwa 60 min). Ungefüllte Zusammensetzungen (siehe Beispiele 1 bis 7 und 11) waren dann einsatzbereit. Für den Fall, dass die Zusammensetzung einen Füllstoff enthält (siehe Beispiele 8 bis 10 und 12 bis 14) wurden diese anschließend mit Hilfe des Rührwerks innerhalb von 15 min untergerührt, danach zweimal mittels eines Drehwalzwerks gewalzt und anschließend nochmals für 15 min gerührt.

**Tabelle 1**: Zusammensetzungen für die Herstellung von z.B. Schienen oder Bohrschablonen

| **Komponente** | **Zusammensetzung [Gew.-%]** | | | | | |
|---|---|---|---|---|---|---|
| | 1 | 2 | 3 | 4 | 5 | 6 |
| TEGDMA[1)] | 23, 0 | 23, 0 | 23, 0 | 23, 0 | 23, 0 | 29, 0 |
| UDMA[2)] | 18,0 | 18, 0 | 18,0 | 18,0 | 18, 0 | 15, 0 |
| bisGMA[3)] | 18,0 | 18,0 | 18,0 | 18,0 | 18,0 | 5, 0 |
| Oligomer (a1)[4)] | - | - | - | - | 40, 0 | 50, 0 |
| Oligomer (a3)[5)] | 40,0 | - | - | - | - | - |
| Oligomer (a5)[6)] | - | 40,0 | - | - | - | - |
| Oligomer (a4)[7)] | - | - | 40,0 | - | - | - |
| Oligomer (a3)[8)] | - | - | - | 40,0 | - | - |
| TPO[9)] | 0,85 | 0,85 | 0,85 | 0,85 | 0,85 | 0,85 |
| Bumetrizol | 0,15 | 0,15 | 0,15 | 0,15 | 0,15 | 0,15 |
| Viskosität (23°C) Ossz. [MPa*s] | 4,8 | 1,3 | 5,2 | - | 5,6 | 3,2 |
| Kmax [MPa $m^{1/2}$] nass | 0,8 | 0,8 | 0,8 | 0,9 | 1,2 | - |
| FW [$J/m^2$] nass | 147 | 114 | 99 | 169 | 189 | - |

[1)] Triethylenglycoldimethacrylat (CAS-Nr. 109-16-0)
[2)] Urethandimethacrylat (CAS-Nr. 72869-86-4)
[3)] Additionsprodukt aus Methacrylsäure und Bisphenol-A-diglycidylether
[4)] Oligomer (a1) mit $R^9$ und $R^{10}$ = H und einer Molmasse von ca. 2200 g/mol
[5)] Oligomer (a3) mit $R^9$ und $R^{10}$ = $CH_3$
[6)] Oligomer (a5) mit $R^9$ und $R^{10}$ = $CH_3$
[7)] Oligomer (a4) mit $R^9$ und $R^{10}$ = $CH_3$
[5)] Oligomer (a3) mit $R^9$ und $R^{10}$ = H
[9)] Diphenyl(2,4,6-trimethylbenzoyl)phenyl-phosphinoxid (CAS-Nr. 75980-60-8)

**Tabelle 2:** Zusammensetzungen für die Herstellung von z.B. Aligner-Attachments tragende Positioner

| Komponente | Zusammensetzung [Gew.-%] | | | | | | | |
|---|---|---|---|---|---|---|---|---|
| | 7 | 8 | 9 | 10 | 11 | 12 | 13 | 14 |
| TEGDMA1) | 25,70 | 23,26 | 20,82 | 18,39 | 25,70 | 23,26 | 20,82 | 18,39 |
| UDMA2) | 13,27 | 12,01 | 10,75 | 9, 49 | 13,27 | 12,01 | 10,75 | 9,49 |
| bisGMA3) | 5,00 | 4,53 | 4,05 | 3,58 | 5,00 | 4,53 | 4,05 | 3,58 |
| Glycerin-1,3-dimethacrylat-2-acetat4) | 10,00 | 9,05 | 8,10 | 7,15 | - | - | - | - |
| Dicyclopentanylmethacrylat5) | - | - | - | - | 10,00 | 9,05 | 8,10 | 7,15 |
| Oligomer (a1)6) | 45,00 | 40,73 | 36,46 | 32,20 | 45,00 | 40,73 | 36,46 | 32,20 |
| TPO7) | 1,00 | 0,91 | 0,81 | 0,72 | 1,00 | 0,91 | 0,81 | 0,72 |
| Bumetrizol | 0,03 | 0,02 | 0,02 | 0,02 | 0,03 | 0,02 | 0,02 | 0,02 |
| Glasfüller8) | - | 4,45 | 8,90 | 13,34 | - | 4,45 | 8,90 | 13,34 |
| Präpolymerfüller9) | - | 5,04 | 10,09 | 15,11 | - | 5,04 | 10,09 | 15,11 |
| Viskosität (23°C) Ossz. [MPa*s] | ~2,0 | 2,0 | 2,9 | 5,0 | 2,0 | 2,1 | 3,1 | 7,0 |
| Biegefestigkeit [MPa] trocken | 89 | 94 | 98 | 104 | 70 | 80 | 95 | 96 |
| Biegemodul [MPa] trocken | 2042 | 2312 | 2655 | 2994 | 1532 | 1957 | 2500 | 2644 |
| Randfaserdehnung bei Bruch [%] trocken | 9,6 | 8,1 | 7,0 | 7,2 | n.g. | n.g. | 11,7 | 9,2 |
| Biegefestigkeit [MPa] nass | 70 | 67 | 72 | 76 | 59 | 59 | 75 | 79 |
| Biegemodul [MPa] nass | 1621 | 1535 | 1886 | 2037 | 1285 | 1462 | 2043 | 2156 |
| Randfaserdehnung bei Bruch [%] nass | 9,8 | 7,2 | 7,8 | 7,5 | n.g. | n.g. | 10,0 | 9,1 |
| Kmax [MPa $m^{1/2}$] trocken | 1,2 | 1,0 | 1,3 | 1,3 | 1,2 | - | - | 1,2 |
| FW [J/$m^2$] trocken | 156 | 97 | 161 | 150 | 173 | - | - | 128 |
| Kmax [MPa $m^{1/2}$] nass | 1,1 | 1,1 | 1,1 | 1,3 | 1,4 | 1,3 | 1,2 | 1,2 |
| FW [J/$m^2$] nass | 189 | 170 | 157 | 206 | 383 | 282 | 182 | 163 |

1) Triethylenglycoldimethacrylat (CAS-Nr. 109-16-0)
2) Urethandimethacrylat (CAS-Nr. 72869-86-4)
3) Additionsprodukt aus Mathacrylsäure und Bisphenol-A-diglycidylether
4) CAS-Nr. 1360927-06-5
5) CAS-Nr. 34759-34-7
6) Oligomer (a1) mit $R^9$ und $R^{10}$ = H und einer Molmasse von ca. 2200 g/mol
7) Diphenyl (2,4,6-trimethylbenzoyl)phenyl-phosphinoxid (CAS-Nr. 75980-60-8)
8) Mischung von einem silanisierten Glasfüller mit einer Partikelgröße von etwa 0,7 µm (vor der Silanisierung) und einem silanisierten Glasfüller mit einer Partikelgröße von etwa 1,0 µm (vor der Silanisierung) in einem Gewichtsverhältnis von etwa 2:1
9) Präpolymerfüllstoff (sog. Isofüller, d.h. ein gemahlenes Kompositmaterial) mit Partikelgrößenverteilung von etwa 0,1 bis etwa 10 µm
n.g.: Prüfkörper nicht gebrochen

Die Zusammensetzungen der Beispiele 1 bis 14 wurden zur additiven Fertigung von dreidimensionalen Bauteilen in einem stereolithographischen Verfahren eingesetzt. Dazu wurden Prüfkörper mittels eines Stereolithographiedruckers im bottomup-Prozess hergestellt. Der Drucker belichtete die Proben in DLP-Technik mit einer Wellenlänge von 388 nm, einer Leistung von 10 mW/$cm^2$ und einer Pixelgröße von 50 µm im schichtweisen Aufbau. Die Schichtdicke betrug jeweils 50 µm.

Die so hergestellten Prüfkörper wiesen eine Dimension von 50 mm Länge, 4 mm Dicke und 8 mm Höhe auf, wobei die Prüfkörper einschließlich einer Einkerbung von 3 mm Tiefe jeweils in der Mitte auf der Oberseite der Prüfkörper

ausgedruckt wurden. Die Einkerbung war jeweils 1 mm breit understreckte sich über die gesamte Dicke der Prüfkörper (4 mm).

Nach dem Ausdruck wurden die Prüfkörper gereinigt und nachvergütet. Anschließend wurde mittels einer Rasierklinge genau in der Mitte der Einkerbung jeweils ein senkrechter Schnitt von ca. 300 $\mu$m Tiefe eingebracht und die Prüfkörper für 24 h bei 37°C in Leitungswasser ("nass") oder an Luft ("trocken") gelagert.

Die Bestimmung der Randfaserdehnung, der Biegefestigkeit und des Biegemoduls erfolgte nach dem oben beschriebenen Verfahren gemäß ISO 4049.

Die Bestimmung der Bruchzähigkeit $K_{max}$ und der Brucharbeit FW erfolgte in Anlehnung an ISO 20795-1:2013 im 3-Punkt Biegeversuch bei einer Stützweite von 32 mm. Die Bestimmung von $K_{max}$ und FW basiert dabei auf den theoretischen Grundlagen des Spannungsintensitätsfaktors $K_{1C}$. Die Bruchzähigkeit $K_{max}$ ist dabei der Höchstfaktor der Beanspruchungsintensität, oder auch Spannungsintensitätsfaktor bei Höchstlast genannt, und wird wie folgt berechnet:

$$K_{max} = \left( \frac{P_{max} \cdot S}{B \cdot W^{\frac{3}{2}}} \right) \cdot f(x) \cdot 0.031 \, \mathrm{MPa} \cdot \mathrm{m}^{1/2}, \text{ mit}$$

$$f(x) = 3 \cdot \sqrt{x} \frac{1.99 - x(1-x) \cdot (2.15 - 3.93x + 2.7x^2)}{2(1+2x) \cdot (1-x)^{\frac{3}{2}}},$$

wobei $x = \frac{a}{W}$

und W die Probenkörperhöhe (= 8 mm), B die Probenkörperdicke (= 4 mm), a die Anrisslänge (= 3mm + Anrisstiefe mit Rasierklinge, S die Stützweite (=32 mm) und $P_{max}$ der maximale Druck bei der Prüfung ist.

Die Berechnung der Brucharbeit (Fracture Work, gesamte Brucharbeit) erfolgte wie folgt:

$$FW = \frac{U}{2B(W-a)} \cdot 1000 \, \mathrm{J/m^2}$$

wobei U die gesamte Energie ist, die für den Bruch der Probe nötig ist (Integral der Last/Weg Grafik) und die gebraucht wird, um die zwei neuen Bruchflächen *B*(*W*-*a*) zu kreieren. Dieser Parameter beschreibt den Widerstand des Materials gegen die Rissausbreitung und ist von den Probeabmessungen und den Testbedingungen abhängig.

Die Prüfkörper zeigten keine visuell wahrnehmbare Eigenfarbe, sondern waren farblos und hochtransparent.

## Patentansprüche

1. Polymerisierbare Zusammensetzung, die

    (a) mindestens eine radikalisch polymerisierbare Verbindung und
    (b) mindestens einen Initiator für die radikalische Polymerisation

    enthält, **dadurch gekennzeichnet, dass** die Zusammensetzung ferner

    (c) mindestens einen UV-Absorber und
    (d) mindestens einen optischen Aufheller

    enthält und als mindestens eine radikalisch polymerisierbare Verbindung (a) mindestens ein radikalisch polymerisierbares Oligomer und mindestens ein radikalisch polymerisierbares, polyfunktionelles Monomer enthält.

2. Polymerisierbare Zusammensetzung nach Anspruch 1, bei der der optische Aufheller (d) eine fluoreszierende Verbindung ist und vorzugsweise eine Verbindung ist, die Licht mit einer Wellenlänge von weniger als 400 nm absorbiert und Licht mit einer Wellenlänge von etwa 400 bis 450 nm emittiert.

3. Polymerisierbare Zusammensetzung nach Anspruch 1 oder 2, bei der der optische Aufheller (d) ausgewählt ist aus der Gruppe bestehend aus 2,5-Bis(5-tert-butyl-benzoxazol-2-yl)thiophen, Terephthalsäurederivaten wie Diethyl-2,5-dihydroxyterephthalat und Mischungen davon.

4. Polymerisierbare Zusammensetzung nach einem der vorhergehenden Ansprüche, bei der der optische Aufheller (d), bezogen auf das Gesamtgewicht der Zusammensetzung, in einer Menge von 0,001 bis 0,1 Gew.-%, vorzugsweise 0,002 bis 0,05 Gew.-%, besonders bevorzugt 0,005 bis 0,02 Gew.-%, vorliegt.

5. Polymerisierbare Zusammensetzung nach einem der vorhergehenden Ansprüche, bei der UV-Absorber (c) ausgewählt ist aus der Gruppe bestehend aus Azofarbstoffen, Carbonylfarbstoffen, Cyaninfarbstoffen, Azomethinen, Methinen, Phthalocyaninen, Dioxazinen, Benzotriazolen, Benzophenonen, Triazinen, Aminstabilisatoren und Mischungen davon.

6. Polymerisierbare Zusammensetzung nach Anspruch 5, bei der der UV-Absorber (c) ausgewählt ist aus der Gruppe bestehend aus 2,2'-Methylenbis[6-(2H-benzotriazol-2-yl)-4-(1,1,3,3-tetramethylbutyl)phenol], 2,2',4,4'-Tetrahydroxybenzophenon, 2-tert-Butyl-6-(5-chloro-2H-benzotriazol-2-yl)-4-methylphenol, 2,2'-Benzol-1,4-diylbis(4h-3,1-benzoxazin-4-on), 2-(4,6-Bis-(2,4-dimethylphenyl)-1,3,5-triazin-2-yl)-5-(octyloxy)-phenol, 2-(2-Hydroxy-5-methylphenyl)benzotriazol, 2-(2-Hydroxyphenyl)benzotriazol, 2-(2H-Benzotriazol-2-yl)-6-dodecyl-4-methylphenol, 2-(2'-Hydroxy-3',5'-di-t-butylphenyl)-5-chlorobenzotriazol, 2,2'-Dihydroxy-4-methoxybenzophenon, 2,2'-Dihydroxy-4,4'-dimethoxybenzophenon, Bis(1,2,2,6,6-pentamethyl-4-piperidyl)sebacat, Methyl-1,2,2,6,6-pentamethyl-4-piperidylsebacat, Bis(1-octyloxy-2,2,6,6-tetramethyl-4-piperidyl)sebacat und Bis(1,2, 2,6,6-pentamethyl-4-piperidyl)-[[3,5-bis(1,1-dimethylethyl)-4-hydroxyphenyl]methyl]butylmalonat und Mischungen davon.

7. Polymerisierbare Zusammensetzung nach Anspruch 5 oder 6, bei der der UV-Absorber (c) 2-tert-Butyl-6-(5-chloro-2H-benzotriazol-2-yl)-4-methylphenol oder 2,2',4,4'-Tetrahydroxybenzophenon ist.

8. Polymerisierbare Zusammensetzung nach einem der vorhergehenden Ansprüche, die, bezogen auf das Gesamtgewicht der Zusammensetzung, UV-Absorber (c) in einer Menge von 0,0001 bis 2,0 Gew.-%, besonders bevorzugt 0,0001 bis 0,5 Gew.-%, enthält, wobei die Zusammensetzung vorzugsweise als UV-Absorber (c) 2-tert-Butyl-6-(5-chloro-2H-benzotriazol-2-yl)-4-methylphenol in einer Menge von 0,01 bis 0,2 Gew.-%, besonders bevorzugt 0,02 bis 0,15 Gew.-%, oder 2,2',4,4'-Tetrahydroxybenzophenon in einer Menge von 0,01 bis 0,07 Gew.-% enthält.

9. Polymerisierbare Zusammensetzung nach einem der vorhergehenden Ansprüche, bei der das Gewichtsverhältnis von UV-Absorber (c) zu optischem Aufheller (d) 2:1 bis 50:1, vorzugsweise 2:1 bis 30:1, besonders bevorzugt 2:1 bis 5:1 oder 10:1 bis 25:1, beträgt.

10. Polymerisierbare Zusammensetzung nach einem der vorhergehenden Ansprüche, bei der das Oligomer ausgewählt ist aus der Gruppe bestehend aus aliphatischen Urethan(meth)acrylat-Oligomeren, Epoxy(meth)acrylat-Oligomeren, Polyether-Urethan(meth)acrylat-Oligomeren und Mischungen davon, wobei das Oligomer vorzugsweise ausgewählt ist aus der Gruppe bestehend aus (a1) einem Oligomer, das durch Polymerisation von 2-Hydroxyethyl(meth)acrylat, 1-Isocyanato-3-isocyanatomethyl-3,5,5-trimethylcyclohexan, ε-Caprolacton und 2-(2-Hydroxyethoxy)ethanol erhältlich ist, (a2) einem Oligomer, das durch Polymerisation von 2-Hydroxyethyl(meth)acrylat, 1-Isocyanato-3-isocyanatomethyl-3,5,5-trimethylcyclohexan, Adipinsäure und Ethylenglykol erhältlich ist, (a3) einem Oligomer, das durch Polymerisation von 2-Hydroxyethyl(meth)acrylat, 1-Isocyanato-3-isocyanatomethyl-3,5,5-trimethylcyclohexan und Tetrahydrofuran erhältlich ist, (a4) einem Oligomer, das durch Polymerisation von 2-Hydroxyethyl(meth)acrylat, 1-Isocyanato-3-isocyanatomethyl-3,5,5-trimethylcyclohexan und Ethylenglykol erhältlich ist, (a5) einem Oligomer, das durch Polymerisation von 2-Hydroxyethyl(meth)acrylat, 2,2,4-Trimethylhexandiol-diisocyanat und Ethylenglykol erhältlich ist, (a6) einem Oligomer, das durch Polymerisation von 2-Hydroxyethyl(meth)acrylat, 2,2,4-Trimethylhexandiol-diisocyanat und Tetrahydrofuran erhältlich ist, und Mischungen davon.

11. Polymerisierbare Zusammensetzung nach Anspruch 10, bei der das Oligomer (a1) ein Oligomer der Strukturformel (I) ist

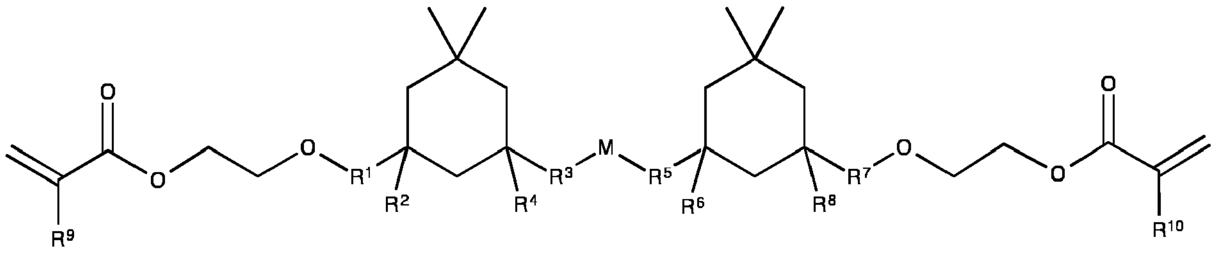

(I),

in der

$R^1$ und $R^5$ jeweils -C(=O)-NH- oder -C(=O)-NH-$CH_2$- sind,
$R^3$ und $R^7$ jeweils -NH-C(=O)- oder $CH_2$-NH-C(=O)- sind,
$R^2$, $R^4$, $R^6$ und $R^8$ jeweils H oder $CH_3$ sind,
$R^9$ und $R^{10}$ jeweils H oder $CH_3$ sind,
wobei,
wenn $R^1$ -C(=O)-NH- ist, dann ist $R^2$ H, R3 -$CH_2$-NH-C(=O)- und $R^4$ $CH_3$, und
wenn $R^1$ -C(=O)-NH-$CH_2$- ist, dann ist $R^2$ $CH_3$, $R^3$ -NH-C(=O)- und $R^4$ H, und
M ein Kettenmittelsegment ist, das durch Polymerisation von ε-Caprolacton und 2-(2-Hydroxyethoxy)ethanol oder durch Polymerisation von 1-Isocyanato-3-isocyanatomethyl-3,5,5-trimethylcyclohexan, ε-Caprolacton und 2-(2-Hydroxyethoxy)ethanol erhältlich ist,
wobei das Oligomer (a1) vorzugsweise ein Oligomer der Strukturformel (II) ist

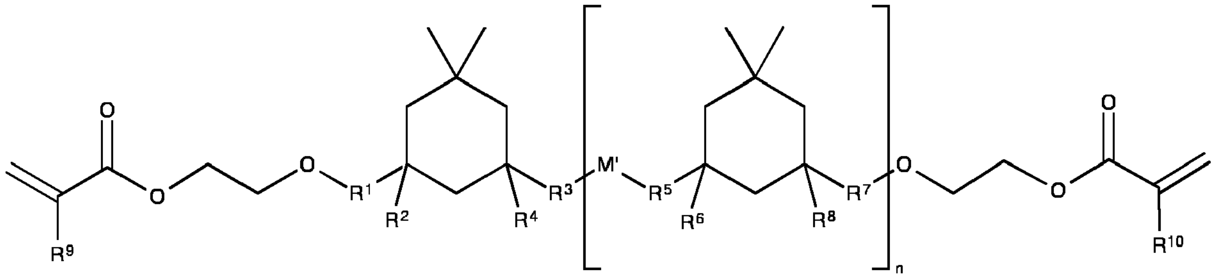

(II),

in der

M' ein Kettenmittelsegment ist, das durch Polymerisation von ε-Caprolacton und 2-(2-Hydroxyethoxy)ethanol erhältlich ist, und
n eine ganze Zahl von 1 bis 10, vorzugsweise 1 bis 7, besonders bevorzugt 1 bis 5, wie etwa 1 bis 4 oder 2 bis 3 und ganz besonders bevorzugt 1 ist,
wobei das Kettenmittelsegment M' der Formel (II) vorzugsweise durch Strukturformel (III) wiedergegeben wird

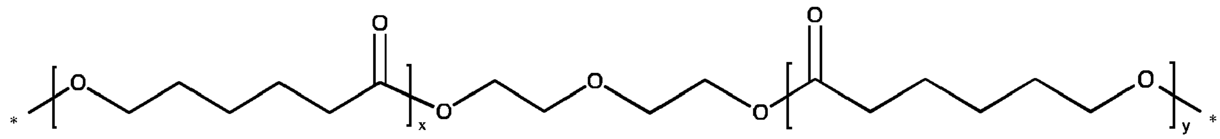

(III),

in der

x eine Zahl von 1 bis 20, vorzugsweise 1 bis 15, besonders bevorzugt 1 bis 5, wie 2 bis 3, ist und
y eine Zahl von 1 bis 20, vorzugsweise 1 bis 15, besonders bevorzugt 1 bis 5, wie 2 bis 3, ist.

**12.** Polymerisierbare Zusammensetzung nach einem der vorhergehenden Ansprüche, bei der das polyfunktionelle Monomer ausgewählt ist aus der Gruppe bestehend aus di- und trifunktionellen Acrylaten und Methacrylaten mit einem Molekulargewicht von <1000 g/mol und deren Mischungen, vorzugsweise aus der Gruppe bestehend aus Glycerin-1,3-dimethacrylat-2-acetat, 2-Phenoxyethyl(meth)acrylat, aliphatischen Urethan-diacrylaten, Phthalsäure-2-Hydroxyethylacrylat-Ester, Pyromellitsäuredi-di-2-Hydroxyethylacrylat-Ester, Bis-G(M)A, 2,2-Bis[4-(2-(meth)-acryloxypropoxy)phenyl]propan, Urethandi(meth)acrylat, Triethylenglycoldi(meth)acrylat, 2-Phenoxethyl(meth)-acrylat, Tricyclodecandimethanoldimethacrylat, und dem Umsetzungsprodukt aus 1,3-Phenylenbis(propan-2,2-diylcarbamoyloxyethan-2,1-diyl)-bis(2-methylacrylat), 2-{[(2-{-[2-Methacryloyloxy)ethoxy]carbonyl}amino)propan-2-yl]phenyl}propan-2-yl)carbamoyl]oxy}propylmethacrylat und 1,3-Phenylenbis(propan-2,2-moyloxypropan-2,1-diyl)bis(2-methylacrylat) sowie deren Mischungen.

**13.** Polymerisierbare Zusammensetzung nach einem der vorhergehenden Ansprüche, bei der der Initiator (c) ausgewählt ist aus der Gruppe bestehend aus Campherchinon, Ethyl-4-(dimethylamino)benzoat, Phenylbis(2,4,6-trimethylbenzoyl)phos-phinoxid, Diphenyl(2,4,6-trimethylbenzoyl)-phenyl-phosphinoxid, 2-Benzyl-2-(dimethylamino)-4'-morpholinobutyrophenon, 1-Butanon-2-(dimethylamino)-2-(4-methylphenyl)methyl-1-4-(4-morpholinyl)phenyl, Acylgermaniumverbindungen und Kombinationen davon, vorzugsweise aus der Gruppe bestehend aus Phenylbis(2,4,6-trimethylbenzoyl)phos-phinoxid, Diphenyl(2,4,6-trimethylbenzoyl)phenyl-phosphinoxid, Bis(4-methoxy-benzoyl)diethylgermanium, Tetrakis(4-ethoxybenzoyl)germanium, Tetrakis(4-propoxybenzoyl)germanium und Kombinationen davon.

**14.** Polymerisierbare Zusammensetzung nach einem der vorhergehenden Ansprüche, die ferner ein monofunktionelles Monomer enthält, vorzugsweise ein monofunktionelles Monomer ausgewählt aus der Gruppe bestehend aus Dicyclopentanylmethacrylat, p-Cumylphenoxyethylenglycolmethacrylat und Mischungen davon.

**15.** Polymerisierbare Zusammensetzung nach einem der vorhergehenden Ansprüche, die ferner bis zu 15 Vol.-% eines Füllstoffs enthält, vorzugsweise eines Füllstoffs mit einer Partikelgröße von weniger als 25 $\mu$m.

**16.** Verwendung einer Zusammensetzung gemäß einem der vorhergehenden Ansprüche als Dentalwerkstoff, insbesondere zur Herstellung oder Reparatur von dentalen Werkstücken.

**17.** Verfahren zur Herstellung oder Reparatur eines dentalen Werkstücks, bei dem eine Zusammensetzung gemäß der Ansprüche 1 bis 15 mit Hilfe von Licht gehärtet wird, um das dentale Werkstück zu ergeben.

**18.** Verwendung gemäß Anspruch 16 oder Verfahren gemäß Anspruch 17, wobei das dentale Werkstück eine Dentalrestauration, eine Prothese, ein Prothesenwerkstoffe, ein künstlicher Zahn, ein Inlay, ein Onlay, eine Krone, eine Brücke, eine Bohrschablone, ein Einprobekörper oder eine orthodontische Vorrichtung ist und vorzugsweise eine orthodontische Vorrichtung ausgewählt aus der Gruppe bestehend aus Alignern, Positionern, Aligner-Attachments, Aligner-Attachments tragenden Positionern, Aufbissschienen, Transferschienen und orthodontischen Schienen ist.

**19.** Verfahren nach Anspruch 17, bei dem die Herstellung oder Reparatur von dentalen Werkstücken durch ein generatives Verfahren, vorzugsweise mit Hilfe von 3D-Druck oder einem Lithographie-basierten Verfahren, wie z.B. Stereolithographie, erfolgt.

Europäisches Patentamt
European Patent Office
Office européen des brevets

## EUROPÄISCHER RECHERCHENBERICHT

Nummer der Anmeldung
EP 18 21 5766

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X | DE 199 07 957 A1 (CIBA GEIGY AG [CH]) 2. September 1999 (1999-09-02) * Ansprüche 1-16 * * Seite 8, Zeile 23 - Seite 9, Zeile 21 * * Seite 10, Zeile 34 - Zeile 43 * * Seite 5, Zeile 66 - Seite 67 * * Seite 6, Zeile 37 - Seite 7, Zeile 44 * * Seite 12, Zeile 14 - Zeile 18 * ----- | 1,3-10, 12-19 | INV. A61K6/09 A61K6/083 |
| A | WO 2016/026915 A1 (IVOCLAR VIVADENT AG [LI]) 25. Februar 2016 (2016-02-25) * Ansprüche 1-15 * ----- | 1-19 | |
| A | US 2018/057691 A1 (LI JIFAN [CN] ET AL) 1. März 2018 (2018-03-01) * Ansprüche 1-33 * ----- | 1-19 | |
| A | WO 2013/153183 A2 (IVOCLAR VIVADENT AG [LI]; UNIV WIEN TECH [AT]) 17. Oktober 2013 (2013-10-17) * Ansprüche 1-24 * ----- | 1-19 | |
| | | | RECHERCHIERTE SACHGEBIETE (IPC) |
| | | | A61K |

Der vorliegende Recherchenbericht wurde für alle Patentansprüche erstellt

2

| Recherchenort | Abschlußdatum der Recherche | Prüfer |
|---|---|---|
| Berlin | 18. Juni 2019 | Siatou, Evangelia |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 18 21 5766

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-06-2019

| Im Recherchenbericht angeführtes Patentdokument | Datum der Veröffentlichung | Mitglied(er) der Patentfamilie | Datum der Veröffentlichung |
|---|---|---|---|
| DE 19907957 A1 | 02-09-1999 | DE 19907957 A1 | 02-09-1999 |
| | | ES 2153315 A1 | 16-02-2001 |
| | | IT MI990376 A1 | 27-08-1999 |
| | | JP H11292910 A | 26-10-1999 |
| | | KR 19990072925 A | 27-09-1999 |
| WO 2016026915 A1 | 25-02-2016 | AU 2015306106 A1 | 02-02-2017 |
| | | CA 2955339 A1 | 25-02-2016 |
| | | CN 106659640 A | 10-05-2017 |
| | | EP 2987480 A1 | 24-02-2016 |
| | | JP 2017524020 A | 24-08-2017 |
| | | US 2017224591 A1 | 10-08-2017 |
| | | WO 2016026915 A1 | 25-02-2016 |
| US 2018057691 A1 | 01-03-2018 | CN 107778406 A | 09-03-2018 |
| | | US 9902860 B1 | 27-02-2018 |
| WO 2013153183 A2 | 17-10-2013 | CN 104246606 A | 24-12-2014 |
| | | EP 2751618 A2 | 09-07-2014 |
| | | JP 6438385 B2 | 12-12-2018 |
| | | JP 2015515327 A | 28-05-2015 |
| | | US 2015111176 A1 | 23-04-2015 |
| | | WO 2013153183 A2 | 17-10-2013 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 3150641 A1
- EP 3293215 A1


### In der Beschreibung aufgeführte Nicht-Patentliteratur


- **A. GEBHARDT.** *Vision of Rapid Prototyping, Ber. DGK,* 2006, vol. 83, 7-12
- **A. GEBHARDT.** Generative Fertigungsverfahren. Carl Hanser Verlag, 2007, 77ff
- **A. BEIL.** Fertigung von Mikro-Bauteilen mittels Stereolithographie. VDI-Verlag, 2002, 3ff
- *CHEMICAL ABSTRACTS,* 43048-08-4
- *CHEMICAL ABSTRACTS,* 178884-91-1
- *CHEMICAL ABSTRACTS,* 1219495-43-8
- *CHEMICAL ABSTRACTS,* 138393-21-2
- *CHEMICAL ABSTRACTS,* 34759-34-7
- *CHEMICAL ABSTRACTS,* 82799-44-8
- *CHEMICAL ABSTRACTS,* 75081-21-9
- *CHEMICAL ABSTRACTS,* 10287-53-3
- *CHEMICAL ABSTRACTS,* 75980-60-8
- *CHEMICAL ABSTRACTS,* 10373-78-1
- *CHEMICAL ABSTRACTS,* 162881-26-7
- *CHEMICAL ABSTRACTS,* 119313-12-1
- *CHEMICAL ABSTRACTS,* 119344-86-4
- *CHEMICAL ABSTRACTS,* 103597-45-1
- *CHEMICAL ABSTRACTS,* 131-55-5
- *CHEMICAL ABSTRACTS,* 3896-11-5
- *CHEMICAL ABSTRACTS,* 18600-59-4
- *CHEMICAL ABSTRACTS,* 2725-22-6
- *CHEMICAL ABSTRACTS,* 2440-22-4
- *CHEMICAL ABSTRACTS,* 23328-53-2
- *CHEMICAL ABSTRACTS,* 3864-99-1
- *CHEMICAL ABSTRACTS,* 131-53-3
- *CHEMICAL ABSTRACTS,* 131-54-4
- *CHEMICAL ABSTRACTS,* 41556-26-7
- *CHEMICAL ABSTRACTS,* 82919-37-7
- *CHEMICAL ABSTRACTS,* 129757-67-1
- *CHEMICAL ABSTRACTS,* 63843-89-0
- *CHEMICAL ABSTRACTS,* 52829-07-9
- *CHEMICAL ABSTRACTS,* 124172-53-8
- *CHEMICAL ABSTRACTS,* 65447-77-0
- *CHEMICAL ABSTRACTS,* 167078-06-0
- *CHEMICAL ABSTRACTS,* 106990-43-6
- *CHEMICAL ABSTRACTS,* 70624-18-9
- *CHEMICAL ABSTRACTS,* 82451-48-7
- *CHEMICAL ABSTRACTS,* 136504-96-6
- *CHEMICAL ABSTRACTS,* 191743-75-6
- *CHEMICAL ABSTRACTS,* 7128-64-5
- *CHEMICAL ABSTRACTS,* 5870-38-2
- *CHEMICAL ABSTRACTS,* 109-16-0
- *CHEMICAL ABSTRACTS,* 72869-86-4
- *CHEMICAL ABSTRACTS,* 1360927-06-5